(19) Europäisches Patentamt
European Patent Office
Office européen des brevets

(11) **EP 2 428 164 A2**

(12) **EUROPEAN PATENT APPLICATION**

(43) Date of publication:
**14.03.2012 Bulletin 2012/11**

(51) Int Cl.:
***A61B 6/02*** (2006.01)

(21) Application number: **11180443.1**

(22) Date of filing: **07.09.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **08.09.2010 JP 2010201074**
**08.09.2010 JP 2010201075**
**08.09.2010 JP 2010201076**
**18.08.2011 JP 2011178725**

(71) Applicant: **Fujifilm Corporation**
**Minato-ku**
**Tokyo (JP)**

(72) Inventor: **Yamaguchi, Yoshitaka**
**Ashigarakami-gun,**
**Kanagawa-ken (JP)**

(74) Representative: **Klunker . Schmitt-Nilson . Hirsch**
**Destouchesstraße 68**
**80796 München (DE)**

(54) **Body motion detection device, as well as radiographic imaging apparatus**

(57) In order to achieve accurate detection of a body motion of a subject in the case where the subject may possibly move between imaging operations, such as during long-length imaging, an imaging information obtaining unit (32) obtains imaging information representing imaging conditions and an imaged subject during the imaging operations, and a local motion vector calculation unit 34 calculates local motion vectors in an overlapping area between each two adjacent radiographic images based on the imaging information. A body motion index value calculation unit 36 calculates a body motion index value with using the local motion vectors based on the imaging information. Further, a body motion determination unit 38 determines whether or not there is a body motion with using the body motion index value based on the imaging information.

FIG.9

**Description**

BACKGROUND OF THE INVENTION

Field of the Invention

The present invention relates to body motion detection device and method for detecting a body motion of a subject during imaging from a plurality of radiographic images obtained by carrying out a plurality of imaging operations with respect to the same subject.

Further, the present invention relates to radiographic imaging apparatus and method for obtaining a single long-length image by taking a plurality of radiographic images and combining the radiographic images.

Description of the Related Art

Conventionally, in the medical field, etc., various types of radiation detectors (so-called "Flat Panel Detectors", hereinafter "FPDs"), which record a radiographic image with respect to a subject by receiving radiation transmitted through the subject, have been proposed and put to practical use. Among such FPDs, for example, there are FPDs using a semiconductor, such as amorphous selenium, which generates an electric charge when exposed to radiation. As this type of FPDs, those of the so-called optical reading system and those of the TFT reading system have been proposed.

On the other hand, with respect to simple X-ray imaging, long-length imaging for imaging a long-length area, such as the entire spine or the entire leg, maybe conducted, and the long-length imaging with using the FPD has also been conducted. When the long-length imaging is conducted, an area that can be imaged with the FPD may be narrower than an area of a subject desired to be imaged. In such a case, the position of the FPD is shifted along a predetermined axis of movement so that the FPD receives radiation transmitted through the same subject each time the position is shifted to achieve the long-length imaging. Each time the radiation is applied (each time a radiographic image is recorded), the recorded image is read out from the FPD to obtain image data representing the recorded radiographic image for each reading operation. Thereafter, the obtained pieces of image data of the radiographic images are combined to join the images with one another to provide image data representing a long part of the subject.

In the long-length imaging using the FPD, however, shot intervals are 3 to 5 seconds, and therefore, a body motion of the subject may occur between shots. If there is a body motion of the subject between shots, it is impossible to join the obtained X-ray images properly, and this hinders accurate measurement. Therefore, it is necessary to retake the images. In some cases, the operator first notices that a body motion occurred during imaging when the operator is observing the combined image, and it is inefficient to retake the images after all the images have once been taken. In addition, shots after the occurrence of body motion are of no use and needlessly increase the radiation exposure of the subject.

The problem of body motion may also occur during energy subtraction imaging, where energy subtraction is carried out with using two radiographic images obtained by applying two radiation rays having different energies to a subject based on the fact that the attenuation of radiation transmitted through a subject vary depending on the substances forming the subject, and during temporal subtraction imaging, where a differential image representing a difference between two radiographic images, which are taken at different imaging times, is obtained. Further, the problem of body motion may also occur during tomosynthesis imaging, where images are taken with moving the X-ray tube to apply the X-ray to a subject from different angles, and the thus obtained images are added up to provide an image in which a desired slice plane is emphasized in order to observe an affected part in more detail, or during continuous shooting to obtain a plurality of images by continuously applying radiation to a subject.

In order to address this problem, techniques to detect a body motion of the subject during imaging to stop the imaging or to warn about the body motion have been proposed. For example, Japanese Unexamined Patent Publication No. 2009-240656 proposes a technique which involves detecting a body motion of a subject during imaging with a sensor and making a warning when a body motion is detected. Further, a technique to calculate an amount of body motion of the subject with taking an assembly error of the FPD and installation error of the radiographic imaging apparatus into account is proposed. Specifically, a technique which involves carrying out template matching in an overlapping area between plurality of radiographic images to find local displacement amounts, calculating an average of the displacement amounts as an amount of body motion, and comparing the amount of body motion with a threshold value to detect whether or not there is a body motion is proposed. It should be noted that the techniques disclosed in Japanese Unexamined Patent Publication No. 2009-240656 are to detect a parallel displacement of the subject relative to the imaging area of the FPD as the body motion. Further, Japanese Unexamined Patent Publication No. 2009-240656 proposes a technique to correct for a body motion by nonlinear warping.

However, the body motion varies for each imaging operation depending on the imaging conditions and the imaged subject of the imaging operation. For example, a higher number of images taken and a longer imaging time or a larger imaging interval between the images taken result in a larger body motion. In addition, a larger body motion occurs when the subject is not secured than that when the subject is secured. Further, if the imaged bodypart contains a moving structure, such as the heart, a larger body motion occurs naturally. In contrast, if the imaged body part is the leg, the body motion can be minimized by the will of the patient, who is the subject. If the subject is, for example, a patient brought to a hospital by the emergency or a patient just after a surgery, the condition of the patient is often severe and it is difficult to hold the body of the patient still in a predetermined position. Therefore, depending on the condition of the subject, it may be impossible to hold the body still during imaging, and a large body motion may occur in such a case. Further, a required amount of body motion to be detected may vary depending on the imaged subject.

Therefore, if the detection of body motion and warning is carried out in a fixed manner, as in the technique disclosed in Japanese Unexamined Patent Publication No. 2009-240656, results of the body motion detection may be non-uniform. Specifically, during imaging of the chest, the heart moves. If a large threshold value is set with taking the movement of the heart into account, it may be difficult to detect a body motion when a different part is imaged. Further, in diagnosis of the entire leg, the length of the leg is roughly measured. On the other hand, in diagnosis of the entire spine, not only the length of the spine but also the bend of the spine is measured. Therefore, the required amount of body motion to be detected is smaller for the entire spine than that for the entire leg. However, if a small threshold value is set with taking accuracy of measurement of the entire spine into account, even a small body motion which does not influence the measurement is detected during long-length imaging of the entire leg.

Further, a body motion includes not only a parallel displacement, as disclosed in Japanese Unexamined Patent Publication No. 2009-240656, but also a three dimensional motion, such as a twisting and a forward or backward inclination of the subject relative to the imaging area of the FPD. In addition, the body motion includes a two dimensional motion, such as a rotation of the subject in a plane parallel to the imaging area and an enlargement or reduction of the subject image due to a parallel displacement of the subject in a forward or backward direction. In the technique disclosed in Japanese Unexamined Patent Publication No. 2009-240656, only an amount of displacement in a direction parallel to the imaging area is calculated as the body motion, and therefore the three dimensional motion and the two dimensional motion of the subject are approximated by the parallel displacement. In the case where the three dimensional motion and the two dimensional motion of the subject are approximated by the parallel displacement in this manner, even if there is a large motion of the subject other than the parallel displacement, the result of detection may indicate only a small body motion or no body motion.

On the other hand, if no body motion is detected (i.e., if the body motion is small), nonlinear warping is applied to achieve body motion correction. However, since the result of detection may indicate a small body motion even when there is a large motion of the subject other than the parallel displacement, as described above, the body motion correction is conducted even when there is a large motion including the three dimensional and two dimensional motions. If the body motion correction is conducted when there is a large motion of the subject, the images are excessively corrected and a large distortion is produced, and this may hinder accurate diagnosis.

Further, in the above-described technique disclosed in Japanese Unexamined Patent Publication No. 2009-240656, a warning is made when a body motion occurs, and the operator retakes the images when the warning is made. However, it is impossible to tell how the body motion occurred only from the warning, and a similar body motion may occur again during the retake operation. If the body motion occurs again during the retake operation, it is necessary to further retake the images, resulting in inefficient imaging and needless increase of the radiation dose of the subject.

SUMMARY OF THE INVENTION

In view of the above-described circumstances, the present invention is directed to achieving accurate detection of a body motion of a subject in the case where the subject may possibly move between imaging operations, such as during long-length imaging.

The present invention is also directed to preventing occurrence of a body motion during a retake operation, which is conducted after a body motion has occurred in the previous imaging operation, during long-length imaging.

A first aspect of a body motion detection device according to the invention includes: image obtaining means for obtaining a plurality of radiographic images taken by carrying out a plurality of imaging operations with respect to an identical subject, the radiographic images at least partially overlapping with one another; imaging information obtaining means for obtaining imaging information representing imaging conditions and an imaged subject during the imaging operations; and body motion index value obtaining means for obtaining a body motion index value based on the imaging information, the body motion index value indicating a body motion of the subject during imaging of the radiographic images.

The description "at least partially overlapping" refers not only to the case where the radiographic images partially overlap with one another, but also refers to the case where the radiographic images entirely overlap with one another.

In the first aspect of the body motion detection device according to the invention, the body motion index value obtaining means may include: local motion vector calculating means for calculating at least one local motion vector representing a local displacement of the subject in an overlapping area between the radiographic images; and body motion index value calculating means for calculating the body motion index value based on the local motion vector.

In this case, the body motion index value calculating means may calculate an index value of an amount of parallel displacement of the subject as the body motion index value.

The body motion index value calculating means may further calculate at least one of an index value of an amount of three dimensional movement of the subject and an index value of an amount of two dimensional movement of the subject as the body motion index value.

The first aspect of the body motion detection device according to the invention may further include body motion determining means for determining whether or not there is a body motion of the subject based on the imaging information and the body motion index value.

In the first aspect of the body motion detection device according to the invention, the imaging information may include at least one of an imaging time during the imaging operations, an imaging interval between the radiographic images, whether or not the subject is immobilized during the imaging operations, an imaged body part of the subject and symptoms of the subject.

A second aspect of the body motion detection device according to the invention includes: image obtaining means for obtaining a plurality of radiographic images taken by carrying out a plurality of imaging operations with respect to an identical subject, the radiographic images at least partially overlapping with one another; imaging information obtaining means for obtaining imaging information representing imaging conditions and an imaged subject during the imaging operations; body motion index value obtaining means for obtaining a body motion index value, the body motion index value indicating a body motion of the subject during imaging of the radiographic images; and body motion determining means for determining whether or not there is a body motion of the subject based on the imaging information and the body motion index value.

In the second aspect of the body motion detection device according to the invention, the body motion index value obtaining means may include: local motion vector calculating means for calculating at least one local motion vector representing a local displacement of the subject in an overlapping area between the radiographic images; and body motion index value calculating means for calculating the body motion index value based on the local motion vector.

In this case, the body motion index value calculating means may calculate an index value of an amount of parallel displacement of the subject as the body motion index value.

The body motion index value calculating means may further calculate at least one of an index value of an amount of three dimensional movement of the subject and an index value of an amount of two dimensional movement of the subject as the body motion index value.

In the second aspect of the body motion detection device according to the invention, the imaging information may include at least one of an imaging time during the imaging operations, an imaging interval between the radiographic images, whether or not the subject is immobilized during the imaging operations, an imaged body part of the subject and symptoms of the subject.

A first aspect of the radiographic imaging apparatus according to the invention is a radiographic imaging apparatus for obtaining a plurality of radiographic images at least partially overlapping with one another by shifting a position of a radiation detector and applying radiation transmitted through a subject to the radiation detector each time the position is shifted, the apparatus including: imaging means for shifting the position of the radiation detector along a predetermined axis of movement, and applying radiation transmitted through the subject to the radiation detector each time the position is shifted; radiographic image obtaining means for obtaining a plurality of radiographic images of the subject by reading out a signal from the radiation detector each time the position is shifted and the radiation is applied; and the body motion detection device of the first or second aspect.

A first aspect of the body motion detection method according to the invention includes: obtaining a plurality of radiographic images taken by carrying out a plurality of imaging operations with respect to an identical subject, the radiographic images at least partially overlapping with one another; obtaining imaging information representing imaging conditions and an imaged subject during the imaging operations; and obtaining a body motion index value based on the imaging information, the body motion index value indicating a body motion of the subject during imaging of the radiographic images.

A second aspect of the body motion detection method according to the invention includes: obtaining a plurality of radiographic images taken by carrying out a plurality of imaging operations with respect to an identical subject, the radiographic images at least partially overlapping with one another; obtaining imaging information representing imaging conditions and an imaged subject during the imaging operations; obtaining a body motion index value indicating a body motion of the subject during imaging of the radiographic images; and determining whether or not there is a body motion of the subject based on the imaging information and the body motion index value.

It should be noted that the first and second aspects of the body motion detection method according to the invention may be provided in the form a program for causing a computer to carry out the method.

According to the first aspect of the body motion detection device and method of the invention, the imaging

4

information representing imaging conditions and an imaged subject during the imaging operations is obtained, and the body motion index value indicating a body motion of the subject during imaging of the radiographic images is obtained based on the imaging information. Therefore, even when the imaging conditions and the imaged subject are changed, the body motion index value can be accurately obtained depending on the imaging conditions and the imaged subject, thereby achieving accurate detection of the body motion.

Further, according to the second aspect of the body motion detection device and method of the invention, the imaging information representing imaging conditions and an imaged subject during the imaging operations are obtained, and whether or not there is a body motion is determined based on the imaging information. Therefore, even when the imaging conditions and the imaged subject are changed, whether or not there is a body motion can be accurately determined depending on the imaging conditions and the imaged subject.

Further, the body motion index value can be obtained by relatively simple calculations by calculating the local motion vector representing a displacement of the subject in the overlapping area for each local area in the overlapping area of the radiographic images, and calculating the body motion index value based on the local motion vectors.

In the case where the index value of an amount of parallel displacement of the subject is calculated as the body motion index value, detection of a body motion attributed to the parallel displacement of the subject is achieved.

In the case where at least one of the index value of an amount of three dimensional movement of the subject and the index value of an amount of two dimensional movement of the subject is calculated as the body motion index value, detection of a three dimensional body motion, such as a twisting and an inclination, of the subject and a two dimensional body motion, such as a rotational movement and enlargement or reduction, of the subject is achieved.

When the first and second aspects of the body motion detection device and method according to the invention is applied to long-length imaging, accurate detection of a body motion of the subject occurring between radiographic images obtained by the long-length imaging is achieved.

A third aspect of the body motion detection device according to the invention includes: image obtaining means for obtaining a plurality of radiographic images taken by carrying out a plurality of imaging operations with respect to an identical subject, the radiographic images at least partially overlapping with one another; and body motion index value obtaining means for obtaining different types of body motion index values according to types of motion of the subject during imaging of the radiographic images.

In the third aspect of the body motion detection device according to the invention, the body motion index value obtaining means may include: local motion vector calculating means for calculating at least one local motion vector representing a local displacement of the subject in an overlapping area between the radiographic images; and body motion index value calculating means for calculating the different types of bodymotion index values based on the local motion vector.

In this case, the body motion index value calculating means may calculate at least two of an index value of an amount of parallel displacement of the subject, an index value of an amount of three dimensional movement of the subject and an index value of an amount of two dimensional movement of the subject as the body motion index values.

The third aspect of the body motion detection device according to the invention may further include post-processing selecting means for selecting post-processing with respect to the radiographic images based on the different types of body motion index values or a primary body motion index value among the different types of body motion index values.

In the third aspect of the body motion detection device according to the invention, the post-processing selecting means may determine a type of the motion of the subj ect for which a body motion is detected based on the different types of body motion index values or the primary body motion index value, and select the post-processing depending on a result of the determination.

In the third aspect of the body motion detection device according to the invention, the post-processing selecting means may select whether or not to apply body motion correction to the radiographic images.

In the third aspect of the body motion detection device according to the invention, the post-processing selecting means may select a type of body motion correction to be applied to the radiographic images.

In the third aspect of the body motion detection device according to the invention, the post-processing selecting means may select a type of body motion index value to be displayed.

A second aspect of the radiographic imaging apparatus according to the invention is a radiographic imaging apparatus for obtaining a plurality of radiographic images at least partially overlapping with one another by shifting a position of a radiation detector and applying radiation transmitted through a subject to the radiation detector each time the position is shifted, the apparatus including: imaging means for shifting the position of the radiation detector along a predetermined axis of movement, and applying radiation transmitted through the subject to the radiation detector each time the position is shifted; radiographic image obtaining means for obtaining a plurality of radiographic images of the subject by reading out a signal from the radiation detector each time the position is shifted and the radiation is applied; and the body motion detection device of the third aspect according to the invention.

A third aspect of the body motion detection method according to the invention includes: obtaining a plurality of radiographic images taken by carrying out a plurality of imaging operations with respect to an identical subject, the radiographic images at least partially overlapping with one another; and obtaining different types of body motion index values according to types of motion of the subject during imaging of the radiographic images.

The third aspect of the body motion detection method according to the invention may be provided in the form of a program for causing a computer to carry out the method.

According to the third aspect of the body motion detection device and method of the invention, different types of body motion index values are obtained according to types of body motion of the subject during imaging of the radiographic images. Therefore, appropriate body motion index values can be obtained according to the body motion of the subject, thereby achieving accurate body motion detection. Further, appropriate selection of the post-processing with respect to the radiographic images to be conducted in a later stage is achieved based on the detected body motion index values.

Further, the body motion index value can be obtained by relatively simple calculations by calculating the local motion vector representing a displacement of the subject in the overlapping area for each local area in the overlapping area of the radiographic images, and calculating the body motion index values based on the local motion vectors.

In the case where at least two of the index value of an amount of parallel displacement of the subject, the index value of an amount of three dimensional movement of the subject and the index value of an amount of two dimensional movement of the subject are calculated as the body motion index values, detection of at least two of a body motion attributed to the parallel displacement of the subject, a three dimensional body motion, such as twisting and inclination, of the subj ect, and a two dimensional body motion, such as rotational movement and enlargement or reduction, of the subject is achieved.

When the third aspect of the body motion detection device and method according to the invention is applied to long-length imaging, accurate detection of a body motion of the subject occurring between radiographic images obtained by the long-length imaging is achieved.

A third aspect of the radiographic imaging apparatus according to the invention is a radiographic imaging apparatus for obtaining a plurality of radiographic images at least partially overlapping with one another by shifting a position of a radiation detector and applying radiation transmitted through a subject to the radiation detector each time the position is shifted, the apparatus including: imaging means for shifting the position of the radiation detector along a predetermined axis of movement, and applying radiation transmitted through the subject to the radiation detector each time the position is shifted; radiographic image obtaining means for obtaining a plurality of radiographic images of the subject by reading out a signal from the radiation detector each time the position is shifted and the radiation is applied; body motion detecting means for detecting a body motion of the subject during imaging of the radiographic images; and information generating means for generating retake assisting information for assisting retake of the radiographic images if the body motion is detected.

The third aspect of the radiographic imaging apparatus according to the invention may further include display means for displaying the retake assisting information.

The third aspect of the radiographic imaging apparatus according to the invention may further include retake controlling means for controlling the retake based on the retake assisting information.

In the third aspect of the radiographic imaging apparatus according to the invention, the retake controlling means may carry out frame allocation for taking the radiographic images with avoiding a position of the subject where the body motion occurred.

In the third aspect of the radiographic imaging apparatus according to the invention, the retake controlling means may increase tightness of a fastener securing the subject.

In the third aspect of the radiographic imaging apparatus according to the invention, the body motion detecting means may include: local motion vector calculating means for calculating at least one local motion vector representing a local displacement of the subject in an overlapping area between the radiographic images; body motion index value calculating means for calculating the body motion index value based on the local motion vector; and body motion determining means for determining whether or not there is the body motion based on the body motion index value.

In this case, the body motion index value calculating means may calculate an index value of an amount of parallel displacement of the subject as the body motion index value.

Further, in this case, the body motion index value calculating means may further calculate at least one of an index value of an amount of three dimensional movement of the subject and an index value of an amount of two dimensional movement of the subject as the body motion index value.

In the third aspect of the radiographic imaging apparatus according to the invention, the body motion detecting means may be a sensor for detecting a motion of the subject.

A radiographic imaging method according to the invention is a radiographic imaging method for obtaining a plurality of radiographic images at least partially overlapping with one another by shifting a position of a radiation'detector and applying radiation transmitted through a subject to the radiation detector each time the position is shifted, the method including the steps of: shifting the position of the radiation detector along a predetermined axis of movement, and applying radiation transmitted through the subject to the radiation detector each time the position is shifted; obtaining a plurality of radiographic images of the subject by reading out a signal from the radiation detector each time the position is shifted and the radiation is applied; detecting a body motion of the subject during imaging of the radiographic images; and generating retake assisting information for assisting retake of the radiographic images if the body motion is detected.

The radiographic imaging method according to the invention may be provided in the form of a program for causing a computer to carry out the method.

According to the third aspect of the radiographic imaging apparatus of the invention and the radiographic imaging method of the invention, the retake assisting information is generated if a body motion is detected. Therefore, the operator can conduct the retake operation according to the retake instruction information so that no body motion occurs. This allows efficiently conducting the retake operation and reducing the exposure dose of the subject.

In particular, in the case where the retake assisting information is displayed, the operator can check the retake assisting information at a glance, and thus can conduct the retake operation more efficiently.

In the case where the retake operation is controlled based on the retake assisting information, the retake operation can be conducted so that no body motion occurs without troubling the operator.

Further, the body motion can be detected by relatively simple calculations by calculating the local motion vector representing a displacement of the subject in the overlapping area for each local area in the overlapping area of the radiographic images, calculating the body motion index value based on the local motion vectors, and determining whether or not there is a body motion based on the body motion index value.

In the case where the index value of the amount of parallel displacement of the subject is calculated as the body motion index value, detection of a body motion attributed to the parallel displacement of the subject is achieved.

In the case where at least one of the index value of the amount of three dimensional movement of the subject and the index value of the amount of two dimensional movement of the subject is calculated as the body motion index value, detection of a three dimensional body motion, such as a twisting and an inclination, of the subject and a two dimensional body motion, such as a rotational movement and enlargement or reduction, of the subject is achieved.

BRIEF DESCRIPTION OF THE DRAWINGS

FIG. 1 is a schematic diagram illustrating the configuration of a radiographic imaging apparatus, to which a body motion detection device according to a first embodiment of the present invention is applied,
FIG. 2 is a diagram for explaining calculation of a local motion vector,
FIG. 3 is a diagram for explaining template matching using multi-resolution conversion,
FIG. 4 is a diagram for explaining another method of template matching,
FIG. 5 is a diagram for explaining yet another method of template matching,
FIG. 6 is diagram illustrating an example of histogram,
FIG. 7 is a diagram for explaining calculation of a body motion index value of a three dimensional motion,
FIG. 8 is a diagram for explaining body motion correction,
FIG. 9 is a flow chart illustrating a process carried out in the first embodiment,
FIG. 10 is a diagram illustrating an example of display on a display screen of an image display unit in the first embodiment,
FIG. 11 is a diagram illustrating another example of the display on the display screen of the image display unit in the first embodiment,
FIG. 12 is a schematic diagram illustrating the configuration of a radiographic imaging apparatus, to which a body motion detection device according to a second embodiment of the invention is applied,
FIG. 13 is a flow chart illustrating a process carried out in the second embodiment,
FIG. 14 is a schematic diagram illustrating the configuration of a radiographic imaging apparatus, to which a body motion detection device according to a third embodiment of the invention is applied,
FIG. 15 is a flow chart illustrating a process carried out in the third embodiment,
FIG. 16 is a diagram illustrating an example of display on the display screen of the image display unit in the third embodiment,
FIG. 17 is diagram illustrating another example of display on the display screen of the image display unit in the third embodiment,
FIG. 18 is a schematic diagram illustrating the configuration of a radiographic imaging apparatus, to which a body motion detection device according to a fourth embodiment of the invention is applied,
FIG. 19 is a flow chart illustrating a process carried out in the fourth embodiment,
FIG. 20 is a diagram illustrating a state where a plurality of combined images are displayed,
FIG. 21 is a diagram illustrating a state where a plurality of combined images are displayed,

FIG. 22 is a schematic diagram illustrating the configuration of a radiographic imaging apparatus according to a fifth embodiment of the invention,

FIG. 23 is flow chart illustrating a process carried out in the fifth embodiment,

FIG. 24 is a diagram illustrating an example of display on the display screen of the image display unit when there is a body motion,

FIG. 25 is a diagram illustrating another example of display on the display screen of the image display unit when there is a body motion,

FIG. 26 is a diagram illustrating yet another example of display on the display screen of the image display unit when there is a body motion,

FIG. 27 is a diagram illustrating still another example of display on the display screen of the image display unit when there is a body motion,

FIG. 28 is a schematic diagram illustrating the configuration of a radiographic imaging apparatus according to a sixth embodiment of the invention,

FIG. 29 is a flow chart illustrating a process carried out in the sixth embodiment, and

FIG. 30 is a flow chart illustrating a process carried out in a seventh embodiment.

DESCRIPTION OF THE PREFERRED EMBODIMENTS

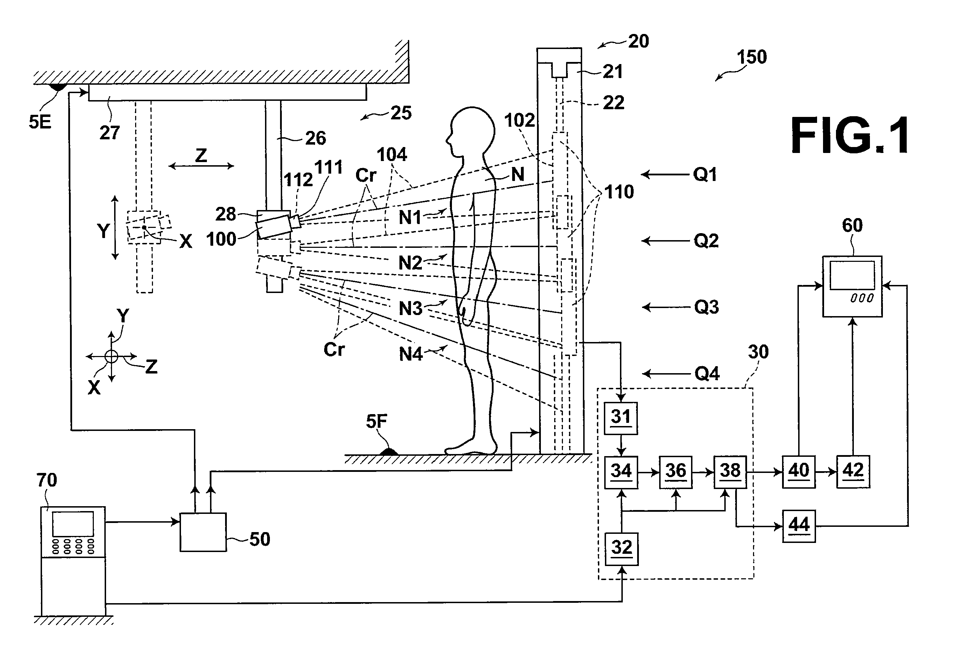

Hereinafter, embodiments of the present invention will be described with reference to the drawings. FIG. 1 is a schematic diagram illustrating the configuration of a radiographic imaging apparatus, to which a body motion detection device according to a first embodiment of the invention is applied. As shown in FIG. 1, a radiographic imaging apparatus 150 according to the first embodiment is adapted to be able to carry out long-length imaging by sequentially imaging a plurality of adjacent areas N1, N2, ... of a subject N with using a single radiation source 100 and a single FPD 110 and combining the thus obtained radiographic images to provide a long-length radiographic image, which represents a major part of the subject N.

It should be noted that the radiographic imaging apparatus 150 according to this embodiment is usable not only for the long-length imaging but also for usual imaging of only a certain part, such as the chest or the leg, of the subject N. In this case, it is possible to carry out energy subtraction imaging, temporal subtraction imaging, etc. However, in the following description, the present invention is described in detail only in relation to the long-length imaging.

Specifically, the radiographic imaging apparatus 150 includes: a radiation source 100 for emitting radiation 104 through a light exit window 111 to an exposure range, which is defined by a collimator 112; a FPD 110 having an imaging area (radiation detector plane) 102 for receiving the radiation 104 transmitted through the subject N to detect the radiation 104; a detector moving unit 20 for moving the FPD 110 along the subject N; and a radiation source positioning unit 25 for positioning the radiation source 100 to position and orient the light exit window 111 in a desired state. In FIG. 1, Cr denotes the radiation central axis of the radiation 104 in the exposure range defined by the collimator 112.

The FPD 110 detects the radiation 104 transmitted through the subject N and converts the detected radiation 104 into an electric signal to output image data representing a radiographic image of the subject N. The FPD 110 may be a direct type FPD, which directly converts the detected radiation into an electric charge, or an indirect type FPD, which once convert the detected radiation into light and further converts the light into an electric signal. The direct type FPD is formed by a photoconductive film, such as amorphous selenium, capacitors and TFTs (Thin Film Transistors) serving as switching devices, etc. For example, when radiation, such as an X-ray, is applied, electron-hole pairs (e-h pairs) are generated at the photoconductive film. The electron-hole pairs are stored in the capacitors, and the electric charges stored in the capacitors are read out via the TFTs as the electric signal.

On the other hand, the indirect type FPD is formed by a scintillator layer made of a fluorescent material, photodiodes, capacitors and TFTs, etc. When radiation, such as "CsI:T1", is applied, the scintillator layer emits light (fluorescence). The fluorescence emitted by the scintillator layer is subjected to photoelectric conversion by the photodiodes and stored in the capacitors, and the electric charges stored in the capacitors are read out via the TFTs as the electric signal.

The detector moving unit 20 includes: two supporting rods 21 standing upright in the vertical direction (the direction of arrow Y in the drawing) from a floor surface 5F for holding the FPD 110 therebetween; and a moving mechanism 22 for moving the FPD 110 in the vertical direction, which is the longitudinal direction. As the moving mechanism 22, one that supports the FPD 110 with a conventionally known linear slide mechanism, or the like, and moves the FPD 110 with using a drive source, such as a motor, may be used.

When imaging operations for obtaining the radiographic images to be combined are conducted, the subject N is positioned along the direction of movement of the FPD 110. That is, the subject N is positioned upright on the floor surface during the imaging operations.

The radiation source positioning unit 25 holds the radiation source 100 to face the imaging area 102 of the FPD 110 via the subject N, i.e., to be oriented toward the FPD 110, and moves the radiation source 100. The radiation source positioning unit 25 includes: a supporting rod 26 extending in the vertical direction from the ceiling 5E; a ceiling base plate 27 for moving the supporting rod 26 along the ceiling 5E in the direction of arrow Z in the drawing; and a rotating mount 28, which is engaged with the supporting rod 26 to be movable in the direction of arrow Y in the drawing and rotatable about an axis that is perpendicular to the plane of the drawing. The radiation source 100 is mounted on the rotating mount 28. Thus, the radiation source 100 is movable in the vertical direction (in the direction of arrow Y in the drawing) and in the right-left direction (in the direction of arrow Z in the drawing), and is rotatable about an axis which is parallel to the X-axis in the drawing and passing through a substantial center of the radiation source 100. The radiation source positioning unit 25 can also be formed by a conventionally known linear slide mechanism, a rotation mechanism and a drive source, such as a motor.

The radiographic imaging apparatus 150 further includes a long-length imaging control unit 50 for controlling operations of the detector moving unit 20 and the radiation source positioning unit 25. The long-length imaging control unit 50 controls operation of the detector moving unit 20 to move the FPD 110 sequentially to positions Q1, Q2, ... for conducting the radiographic imaging operations in the direction along the subject N. In conjunction with this control, the long-length imaging control unit 50 controls operation of the radiation source positioning unit 25 to position the radiation source 100 so that the radiation 104 emitted from the radiation source 100 is directed to the imaging area 102 of the FPD 110, which is sequentially positioned in the above-described positions. When the radiation source 100 is driven in this state, adjacent areas N1, N2, ... of the subject N are sequentially imaged in the individual imaging operations to provide pieces of image data representing partial radiographic images used to form an image of the entire subject N.

The radiographic imaging apparatus 150 further includes: a body motion detection unit 30 for detecting a body motion of the subject N during the imaging operations; a body motion correction unit 40 for correcting for an amount of displacement of the subject N based on the body motion; an image combining unit 42 for combining the pieces of image data obtained by the above-described radiographic imaging operations to generate a long-length radiographic image representing the entire subject N; and a warning unit 44. The long-length radiographic image generated by the image combining unit 42 is displayed on an image display unit 60, which is formed, for example, by a CRT display device, a liquid crystal display device, or the like.

The body motion detection unit 30 includes an image obtaining unit 31, an imaging information obtaining unit 32, a local motion vector calculation unit 34, a body motion index value calculation unit 36 and a body motion determination unit 38. It should be noted that the local motion vector calculation unit 34 and the body motion index value calculation unit 36 form a body motion index value obtaining means.

The image obtaining unit 31 is formed by various interfaces for obtaining the radiographic images from the FPD 110. It should be noted that, in the case where the body motion detection unit 30 is provided separately from the radiographic imaging apparatus 150 and the body motion detection unit 30 is connected to the radiographic imaging apparatus 150 via a network, the image obtaining unit 31 is a network interface.

The imaging information obtaining unit 32 obtains imaging information from a console 70, which controls the entire operation of the radiographic imaging apparatus 150. The imaging information includes information about imaging conditions and information about the imaged subject. The information about imaging conditions may include an imaging time taken for taking all the radiographic images in the long-length imaging, an imaging interval between each two adjacent radiographic images, whether or not the subject N is secured during imaging, etc. The information about the imaged subject includes the imaged body part (such as the chest, the leg, the entire spine, the entire leg, or the like) of the subject N and symptoms of the subject N (such as whether the condition is severe or mild, whether the subj ect is before or after a surgery, etc.) The imaging information obtaining unit 32 obtains at least one of these items as the imaging information.

It should be noted that the imaging information may be inputted by the operator via the console 70, or may be automatically calculated by the console 70 by measuring the imaging time, detecting whether or not the subject N is secured, recognizing the body part captured in the obtained radiographic images, etc., at the console 70. Since the imaging information varies depending on the imaging menu, the console 70 may automatically determine the imaging information depending on the imaging menu selected by the operator.

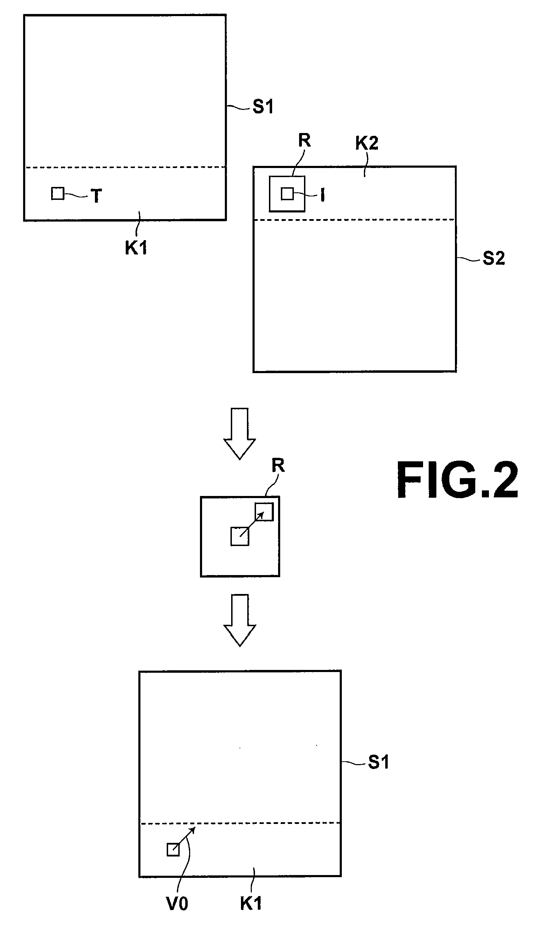

The local motion vector calculation unit 34 calculates a local motion vector in an overlapping area between each two adjacent radiographic images. FIG. 2 is a diagram for explaining the calculation of the local motion vector. As shown in FIG. 2, the local motion vector calculation unit 34 calculates an amount of local movement, i.e., the local motion vector, which appears in the images due to a body motion of the subject N, in overlapping areas K1 and K2 of two adjacent radiographic images S1 and S2 by applying template matching, where a certain image portion in one of the images is used as a template (a template T with a control point present in the overlapping area K1 of the radiographic image S1 being the reference) to find a corresponding image portion in the other of the images (in the overlapping area K2 of the radiographic image S2).

Specifically, the local motion vector calculation unit 34 calculates a correlation value between the template T and each image portion of interest I, which has the same size as the template T and is sequentially searched in the overlapping area K2 of the radiographic image S2, in a predetermined search range R. This operation provides a correlation distribution having the same size as the search range R. Then, an amount of pixel displacement (amount of movement) of a position where the maximum correlation value is found in the correlation distribution from a reference position (a position where the maximum correlation value is found when there is no body motion) of the template T is calculated as a local motion vector V0.

It should be noted that, in this embodiment, a plurality of control points are set. Therefore, a plurality of local motion vectors are calculate in the overlapping area K1. The control points may be all the pixel positions of the image in the overlapping area K1, or may be pixel positions decimated with a predetermined pixel interval in the overlapping area K1. Alternatively, the control points may be points of intersection of edges in the overlapping area K1, or representative pixel positions, such as pixel positions with a large variance. The representative pixel positions may be automatically detected, or may be manually set by the operator on the overlapping area K1 of the radiographic image S1 being displayed.

The local motion vector V0 may be calculated not only based on the position where the maximum correlation value is found but also based on a centroid position of the correlation values in the correlation distribution. The centroid position of the correlation values can be found by calculating a weighted average position based on the correlation values at pixel positions in the search range R with an origin (for example, the reference position of the above-described template) in the search range R being the reference. At this time, the centroid position may be calculated with using only pixel positions where the correlation value is equal to or higher than a predetermined threshold value. This centroid position is referred to as "high correlation centroid position".

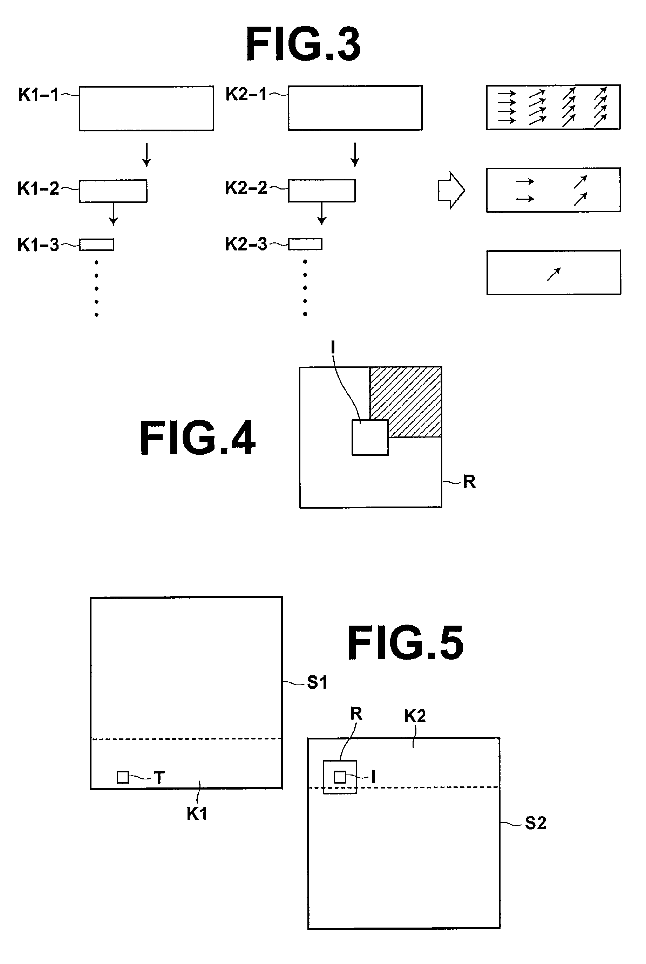

Further, as shown in FIG. 3, when the local motion vectors V0 are calculated, multi-resolution conversion may be applied to the images in the overlapping areas K1 and K2 to generate overlapping area images of different resolutions, and the local motion vectors V0 between the overlapping area images of each resolution may be sequentially calculated in the order from the lowest resolution to the highest resolution. It should be noted that FIG. 3 shows a state where the resolution conversion is conducted twice to generate multi-resolution images of resolutions down to 1/4 resolution. Further, in FIG. 3, the overlapping area images before subjected to the multi--resolution conversion are denoted by K1-1 and K2-1, the overlapping area images of the next resolution are denoted by K1-2 and K2-2, and the overlapping area images of the further next resolution are denoted by K1-3 and K2-3, respectively.

Each resolution conversion of the multi-resolution conversion generates the overlapping area images of a resolution reduced by one half. Therefore, assuming that 16 local motion vectors V0 are calculated in the case where the resolution conversion is applied twice to achieve the multi-resolution conversion down to 1/4 resolution, first, one local motion vector V0 is calculated with using the overlapping area images K1-3 and K2-3 of the lowest resolution. It shouldbe noted that, at a part of FIG. 3 for explaining states where each local motion vector V0 is calculated, the overlapping area images of different resolutions are shown with the same size as the size of the overlapping areas K1-1, K2-1 for convenience.

Subsequently, four local motion vectors V0 are calculated with using the overlapping area images K1-2 and K2-2 of the second highest resolution. At this time, efficient calculation of the four local motion vectors V0 can be achieved by using the local motion vector V0 calculates with using the overlapping area images K1-3 and K2-3. For example, in the case where the local motion vector V0 calculated for the overlapping area images K1-3 and K2-3 is a motion vector directed diagonally to the upper right, as shown in FIG. 3, this local motion vector V0 is used as an initial value with respect to the overlapping area images K1-2 and K2-2 of the next resolution to conduct the template matching only in an area around the upper right area in the search range R (indicated with hatching), as shown in FIG. 4. This can reduce the amount of calculation for calculating the correlation values, thereby achieving efficient calculation of the local motion vectors V0.

Although the search range R is set in the overlapping area K2 for conducting the template matching in the above description, the search range R may be set beyond the overlapping area K2, as shown in FIG. 5. By increasing the size of the search range in this manner, more accurate calculation of the local motion vectors V0 can be achieved.

Further, although the template T is set in the overlapping area K1 of the radiographic image S1 to calculate the local motion vectors V0 in the above description, the template T may also be set in the overlapping area K2 of the radiographic image S2, in addition to the template T set in the overlapping area K1, to calculate the local motion vectors V0 with the overlapping area K2 being the reference.

Still further, although the correlation values are used to calculate the local motion vectors V0 in the above description, any index other than the correlation value, such as a residual error or a mean square error, which indicates the degree of similarity between the template T and each image portion of interest I in the search range R may be used.

As mentioned above, the local motion vector calculation unit 34 can calculate the local motion vectors V0 with using any of various methods; however, in this embodiment, the local motion vector calculation unit 34 calculates the local motion vectors V0 based on the imaging information obtained by the imaging information obtaining unit 32. Specifically, the method used to calculate the local motion vectors V0 is changed depending on the imaging information. For example, if the information about imaging conditions contains information indicating that a large number of images are taken, that the imaging time is long, that the imaging interval is long, and that the subject is not secured, etc., or if the information about the imaged subject contains information indicating that the imaged body part is the chest (in the case other than the long-length imaging) or the entire spine (in the case of the long-length imaging), that the condition of the patient, who is the subject, is severe and it is impossible to keep the body of the patient still during imaging, etc., then it is assumed that there may be a large body motion. Therefore, if it is assumed that there may be a large body motion, the local motion vector calculation unit 34 may set a large search range R and/or apply the multi-resolution conversion to the overlapping areas to generate overlapping area images of resolutions down to even lower resolutions for conducting the template matching. This allows more accurate calculation of the local motion vectors V0. It should be noted that whether or not the imaging time is long and whether or not the imaging interval is long may be determined using threshold values.

In contrast, if the imaging information contains information from which it is assumed that there may be a small body motion, a small search range R may be set and/or the number of times of the multi-resolution conversion may be reduced or the multi-resolution conversion may not be applied. The local motion vector calculation unit 34 may determine the magnitude of the body motion in a stepwise manner, in addition to determining whether or not there is a large body motion. In this case, the size of the search range R may be changed in a stepwise manner and/or the number of times of the multi-resolution conversion may be changed in a stepwise manner depending on the magnitude of the body motion.

Further, if the imaging information contains information of an imaged body part which is supposed to contain many two dimensional structures (such as the chest, the entire spine, or the like), each local motion vector V0 may be calculated based on the position where the maximum correlation value is found during the template matching. In contrast, if the imaging information contains information of an imaged body part which is supposed to contain many one dimensional structures (such as the leg, the entire leg, or the like), the correlation values tend to deviate along the one dimensional direction. Therefore, each local motion vector V0 may be calculated based on the centroid position or the high correlation centroid position.

The body motion index value calculation unit 36 calculates a body motion index value with using the local motion vectors V0 calculated by the local motion vector calculation unit 34. First, the body motion index value calculation unit 36 calculates a body motion index value of a parallel displacement. The parallel displacement refers to a linear motion of the subject N parallel to the imaging area 102 of the FPD 110. Therefore, the body motion index value calculation unit 36 generates a three dimensional histogram, where three axes correspond to a vertical displacement, a horizontal displacement and a frequency, with respect to the local motion vectors V0 calculated by the local motion vector calculation unit 34. The vertical displacement and the horizontal displacement correspond to the vertical and horizontal directions of the overlapping areas of the radiographic images, respectively. Each local motion vector V0 is expressed, with using the coordinate system shown in FIG. 1, by a two dimensional vector on X-Y coordinates where the horizontal direction is represented by the X-coordinate and the vertical direction is indicated by the Y-coordinate. Therefore, the vertical displacement and the horizontal displacement of each local motion vector V0 are represented by the magnitudes in the Y direction and the X direction of the local motion vector V0.

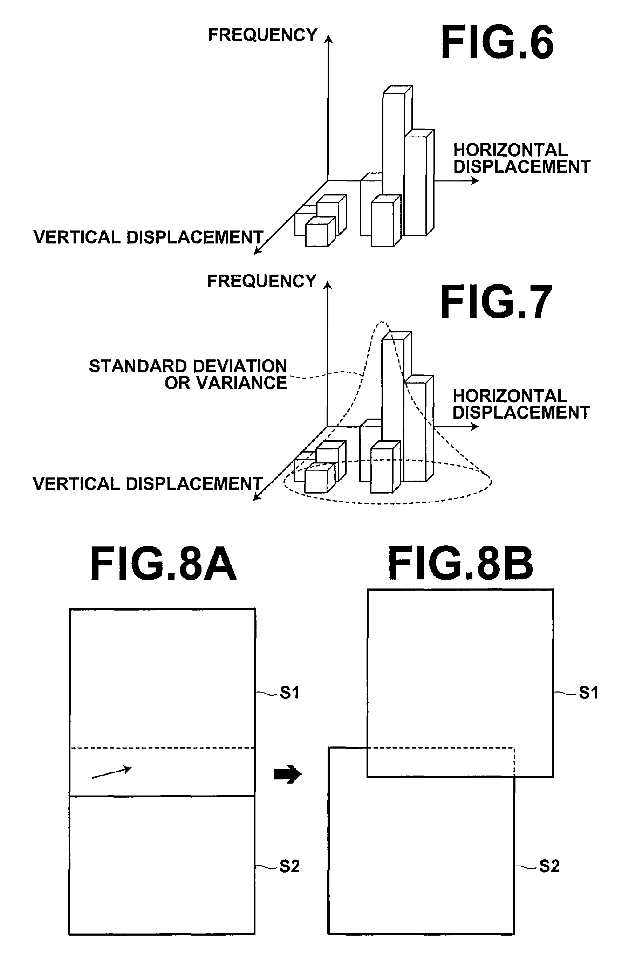

FIG. 6 is a diagram illustrating an example of the histogram. As shown in FIG. 6, this histogram shows frequencies of the local motion vectors V0 depending on the values of the vertical displacement and the horizontal displacement. It should be noted that, for generating the histogram, only the local motion vectors V0 with the correlation value, which serves as the basis of the calculation of the local motion vectors V0, equal to or higher than a predetermined threshold value may be used. Alternatively, variance values of pixel values in the template T used to calculate each local motion vector V0 may be calculated, and only the local motion vectors V0 which are calculated using the template T with a variance value smaller than a predetermined threshold value and containing many edges may be used.

Then, the body motion index value calculation unit 36 calculates the body motion index value based on the histogram. For example, the body motion index value may be calculated by determining the local motion vectors V0 with the maximum frequency in the histogram, and calculating a distance between the position of the local motion vectors V0 on the histogram (maximum frequency position) and a reference position (i.e., the origin of the histogram) as the body motion index value. Alternatively, the body motion index value may be calculated by calculating a centroid position (frequency centroid position) of the local motion vectors V0 with the frequency equal to or higher than a predetermined threshold value, and then, calculating a distance between the frequency centroid position and the reference position as the body motion index value. Further alternatively, an average of the vertical displacements and an average of the horizontal displacements of the local motion vectors V0 may be calculated to calculate an average position of the local motion vectors V0, and a distance between the average position and the reference position may be calculated as the body motion index value. Still alternatively, a median value of the vertical displacements and a median value of the horizontal displacements of the local motion vectors V0 may be calculated to calculate a median position of the local motion vectors V0, and a distance between the median position and the reference position may be calculated as the body motion index value.

Further, the body motion index value calculation unit 36 calculates a body motion index value of a three dimensional motion of the subject N. The three dimensional motion refers to a twisting of the subject N and an inclination of the subject N in the front-back direction relative to the imaging area 102 of the FPD 110. The body motion index value of the three dimensional motion is calculated with using the histogram of the local motion vectors V0 calculated as described above. FIG. 7 is a diagram for explaining the calculation of the body motion index value of the three dimensional motion. It should be noted that, in the case where the body motion includes only a parallel displacement, the distribution on the histogram concentrates substantially at one position. In contrast, in the case where the body motion includes a three dimensional motion, the distribution on the histogram spreads. Therefore, the body motion index value calculation unit 36 calculates a standard deviation or a variance of the distribution on the histogram as the body motion index value of the three dimensional motion. The larger the standard deviation or variance, the larger the three dimensional motion. As the center for calculating the standard deviation or variance, any of the maximum frequency position, the frequency centroid position, the average position and the median position described above may be used.

Further, the body motion index value calculation unit 36 calculates a body motion index value of a two dimensional motion of the subject N. The two dimensional motion refers to a rotation of the subject N in a plane parallel to the imaging area 102 of the FPD 110, a parallel displacement of the subject N in the front-back direction relative to the imaging area 102, and a parallel displacement of the subj ect N in a direction parallel to the imaging area 102. It should be noted that the body motion of the parallel displacement in the front-back direction relative to the imaging area appears as an enlargement or reduction in a radiographic image. Therefore, in the following description, the body motion in the front-back direction is described as a body motion of enlargement/reduction. Further, although the motion of parallel displacement can be calculated with using the local motion vectors V0, as described above, it is assumed in this description of the calculation of the two dimensional motion that the two dimensional motion includes all of the rotation, the enlargement/reduction and the parallel displacement.

The body motion index value calculation unit 36 calculates the body motion index value of the two dimensional motion of the subject N based, for example, on equations disclosed in "Zukei-shori-kogaku (graphic processing engineering)", Fujio Yamaguchi, published by Nikkan Kogyo Shimbun Ltd., pp. 68-82, 1981. Specifically, the local motion vectors V0 calculated as described above are applied to an equation of two-dimensional affine transformation to calculate individual elements of the parallel displacement, the rotation and the enlargement/reduction as the body motion index values from the plurality of local motion vectors V0 with using the least-squares method. Assuming that an amount of movement in the horizontal direction is tx and an amount of movement in the vertical direction is ty in the two dimensional affine transformation, the parallel displacement is expressed by equation (1) below (in equation (1) and the following equations, (x,y) is a two dimensional coordinate position before the body motion and (x*,y*) is a two dimensional coordinate position after the body motion) :

$$(x \; y \; 1) \begin{pmatrix} 1 & 0 & 0 \\ 0 & 1 & 0 \\ tx & ty & 1 \end{pmatrix} = (xt^* \; yt^* \; 1) \qquad (1).$$

Assuming that

$$\bar{I} = (x \; y \; 1)$$

$$Ht = \begin{pmatrix} 1 & 0 & 0 \\ 0 & 1 & 0 \\ tx & ty & 1 \end{pmatrix}$$

$$It^* = (xt^* \; yt^* \; 1),$$

then equation (1) becomes:

$$IHt = It^*  \qquad (1').$$

Further, assuming that a rotational angle is θ in the two dimensional affine transformation, the rotational movement is expressed by equation (2) below:

$$(x \ y \ 1) \begin{pmatrix} cos\theta & sin\theta & 0 \\ -sin\theta & cos\theta & 0 \\ x_{00}(1 - cos\theta) + y_{00}\,sin\theta & y_{00}(1 - cos\theta) - x_{00}\,sin\theta & 1 \end{pmatrix} = (x_\theta^* \ y_\theta^* \ 1) \quad (2),$$

wherein $x_{00}\,y_{00}$ is the center of rotaion.

Assuming that

$$I = (x \ y \ 1)$$

$$H_\theta = \begin{pmatrix} cos\theta & sin\theta & 0 \\ -sin\theta & cos\theta & 0 \\ x_{00}(1 - cos\theta) + y_{0\theta}\,sin\theta & y_{00}(1 - cos\theta) - x_{0\theta}\,sin\theta & 1 \end{pmatrix}$$

$$I_\theta^* = (x_\theta^* \ y_\theta^* \ 1),$$

then equation (2) becomes:

$$IH_\theta = I_\theta^* \qquad (2').$$

Further, assuming that enlargement/reduction parameters for the enlargement/reduction are a (in the X direction) and d (in the Y direction), the enlargement/reduction in the two dimensional affine transformation is expressed by equation (3) below:

$$(x \ y \ 1) \begin{pmatrix} a & 0 & 0 \\ 0 & d & 0 \\ x_{0ad}(1 - a) & y_{0ad}(1 - d) & 1 \end{pmatrix} = (x_{ad}^* \ y_{ad}^* \ 1) \quad (3),$$

wherein $x_{0ad},y_{0ad}$ is the center of rotaion.

Assuming that

$$I = (x \ y \ 1)$$

$$H_{ad} = \begin{pmatrix} a & 0 & 0 \\ 0 & d & 0 \\ x_{0ad}(1-a) & y_{0ad}(1-d) & 1 \end{pmatrix}$$

$$I_{ad}^* = (\ x_{ad}^* \quad y_{ad}^* \quad 1\ ),$$

then equation (3) becomes:

$$IH_{ad} = I_{ad}^* \qquad (3')\,.$$

Combining equations (1') to (3'), equation (4) below is obtained:

$$IH = I^* \qquad (4)$$

$$H = Ht\ H_\theta\ H_{ad}$$

where H is a matrix containing nine elements. The local motion vector V0 can be expressed as (x*-x,y*-y). Therefore, the amount of parallel displacement, the rotational angle and the enlargement/reduction parameters can be calculated by applying the local motion vectors V0 to equation (4) above to find the nine elements with using the least-squares method. The thus calculated amount of parallel displacement, rotational angle and enlargement/reduction parameters are used as the body motion index values of the two dimensional motion. It should be noted that, in the subsequent operations in this embodiment, the body motion index value of the parallel displacement calculated based on the histogram, as described above, is used as the body motion index value of the parallel displacement, and only the rotational angle and the enlargement/reduction parameters are used as the body motion index values of the two dimensional motion.
Although the body motion index value calculation unit 36 can calculate the body motion index values of the parallel displacement and the three dimensional motion with using any of various methods, as described above, the body motion index value calculation unit 36 in this embodiment calculates the body motion index values based on the imaging information obtained by the imaging information obtaining unit 32. Specifically, the methods used to calculate the body motion index values are changed depending on the imaging information. For example, with respect to the body motion index value of the parallel displacement, if the imaged body part is the leg (in the case other than the long-length imaging) or the entire leg (in the case of the long-length imaging), there is no structure that moves independently from the body motion of the subject N, such as the heart or gas in the intestine. Therefore, if the imaging information contains information indicating that the imaged body part is the leg, then the body motion index value is calculated with using the average position and the median position, thereby calculating the index value with using the local motion vectors V0 as many as possible. With respect to the body motion index value of the three dimensional motion, the body motion index value is calculated with using the average position and the median position as the center for calculating the standard deviation or variance.
On the other hand, if the imaged body part is the chest (in the case other than the long-length imaging) or the entire spine (in the case of the long-length imaging), there is a structure that moves independently from the body motion of the subject N, such as the heart or gas in the intestine. Therefore, if the imaging information contains information indicating that the imaged body part is the chest, the body motion index value of the parallel displacement is calculated with using the maximum frequency position or the frequency centroid position, thereby calculating the index value without being influenced by a local motion. With respect to the body motion index value of the three dimensional motion, the body motion index value is calculated with using the maximum frequency position or the frequency centroid position as the center for calculating the standard deviation or variance.
Although the body motion index value calculation unit 36 calculates all the body motion index values of the parallel displacement, the three dimensional motion and the two dimensional motion in this example, the body motion index value calculation unit 36 may calculate at least one of the body motion index values of the parallel displacement, the three dimensional motion and the two dimensional motion. It is preferred that the body motion index value of the parallel displacement is included.

The body motion determination unit 38 compares the body motion index value calculated by the body motion index value calculation unit 36 with a threshold value, and if the body motion index value is equal to or higher than the threshold value, the body motion determination unit 38 determines that there is a body motion and outputs the result of determination. It should be noted that, if two or more of the body motion index values of the parallel displacement, the three dimensional motion and the two dimensional motion are calculated, the body motion determination unit 38 compares each body motion index value with a corresponding threshold value to determine whether or not there is a body motion. In this case, if any one of the body motion index values is equal to or higher than the threshold value, it is determined that there is a body motion. Alternatively, it may be determined that there is a body motion if any two of the body motion index values are equal to or higher than the threshold values, or all the body motion index values are equal to or higher than the threshold values. Further, whether or not there is a body motion may be determined for each of the parallel displacement, the three dimensional motion and the two dimensional motion. Still further, with respect to the two dimensional motion, a threshold value for the rotation and a threshold value for the enlargement/reduction may be prepared, and whether or not there is a body motion may be determined for each of the rotation and the enlargement/reduction.

In this embodiment, the body motion determination unit 38 determines whether or not there is a body motion by comparing the body motion index value with the threshold value based on the imaging information calculated by the imaging information obtaining unit 32. Specifically, the magnitude of the threshold value is changed depending on the imaging information. For example, if the imaged body part is the leg (in the case other than the long-length imaging) or the entire leg (in the case of the long-length imaging), there is no structure that moves independently from the body motion of the subject N, such as the heart or gas in the intestine. In contrast, if the imaged body part is the chest (in the case other than the long-length imaging) or the entire spine (in the case of the long-length imaging), there is a structure that moves independently from the body motion of the subject N, such as the heart or gas in the intestine. Therefore, if the imaging information contains information indicating that the imaged body part is the chest, a larger threshold value than that used in the case where the imaged body part is the leg is used for the determination. In this manner, the determination as to whether or not there is a body motion can be achieved without being influenced by a local motion.

Further, if the imaged body part is one that requires detection of a smaller amount of body motion, such as the entire spine (in the case of the long-length imaging), a smaller threshold value is set. In contrast, if the imaged body part is one that requires detection of a larger amount of body motion, such as the entire leg (in the case of the long-length imaging), a larger threshold value is set.

Next, body motion correction carried out by the body motion correction unit 40 is described. If the result of determination by the body motion determination unit 38 is that there is no body motion, then the body motion correction unit 40 corrects the radiographic images to eliminate image distortion due to a body motion of the subject N based on the body motion index value detected by the body motion detection unit 30. In contrast, if the result of determination is that there is a body motion, no body motion correction is conducted since the images would be excessively distorted if the body motion correction is conducted. Now, correction for eliminating image distortion between two adjacent radiographic images is described. As shown at "a" in FIG. 8, in the case where the body motion index value of the parallel displacement is detected, the body motion correction is achieved by shifting two radiographic images S1 and S2 relative to each other, as shown at "b" in FIG. 8, based on the body motion index value, i.e., the local motion vectors V0. In the case where two or more body motion index values are detected, the body motion correction is achieved by nonlinearly warping the radiographic images relative to each other so that the template and the corresponding image portion of interest, for which the amount of displacement has been detected, are aligned with each other, that is, the positions of the local motion vectors V0 are aligned between the two images.

In the case where all the body motion index values of the parallel displacement, the three dimensional motion and the two dimensional motion are calculated, the body motion correction may be achieved by conducting nonlinear warping if all the body motion index values are not 0 (that is, there is a body motion including at least two of the parallel displacement, the three dimensional motion and the two dimensional motion) . If only one of the body motion index values of the parallel displacement, the three dimensional motion and the two dimensional motion is not 0, the body motion correction depending on the motion may be conducted. For example, if only the body motion index value of the parallel displacement is not 0, the body motion correction may be achieved by shifting the radiographic images relative to each other. If only the body motion index value of the three dimensional motion is not 0, the body motion correction may be achieved by the nonlinear warping. If only the body motion index value of the two dimensional motion is not 0, the body motion correction may be achieved by the two dimensional affine transformation using the body motion index values (i.e., the elements of the rotation and the enlargement/reduction except for the parallel displacement in equations (1) to (3)). If only one of the body motion index values of the rotation and the enlargement/reduction, among the body motion index values of the two dimensional motion, is not 0, the body motion correction may be achieved by the two dimensional affine transformation using the body motion index value that is not 0. If there is a body motion of the two dimensional motion, the nonlinear warping may be conducted. Further, the body motion correction may be conducted depending on a motion with the largest body motion index value among the body motion index values of the three dimensional motion and the two dimensional motion.

The image combining unit 42 combines the radiographic images, which have been subjected to the body motion correction, by joining the images to generate a combined image C1.

The warning unit 44 makes a warning if there is a large body motion, as will be described later.

The entire operation of the radiographic imaging apparatus 150 is controlled by the console 70. Therefore, information about the subject N, imaging conditions for obtaining the long-length radiographic image, etc., are inputted to the console 70, and these pieces of information are inputted to the long-length imaging control unit 50 and an imaging adjusting unit (not shown) for setting the radiation exposure range defined by the collimator 112, etc. The imaging adjusting unit carries out frame allocation for adjusting the position of the radiation source 100, the condition of the collimator 112, the position of the FPD 110, etc., during each radiation imaging operation so that a radiographic image to be combined of a predetermined size is obtained by each of, for example, four radiographic imaging operations. Thereafter, the operations to take four radiographic images are conducted according to a command fed from the console 70.

To determine the sizes of the radiographic images taken by the imaging operations, a part of the radiographic image obtained by each imaging operation may be cut out to adjust the length and width of the image part, besides defining the radiation exposure range with the collimator 112, as described above.

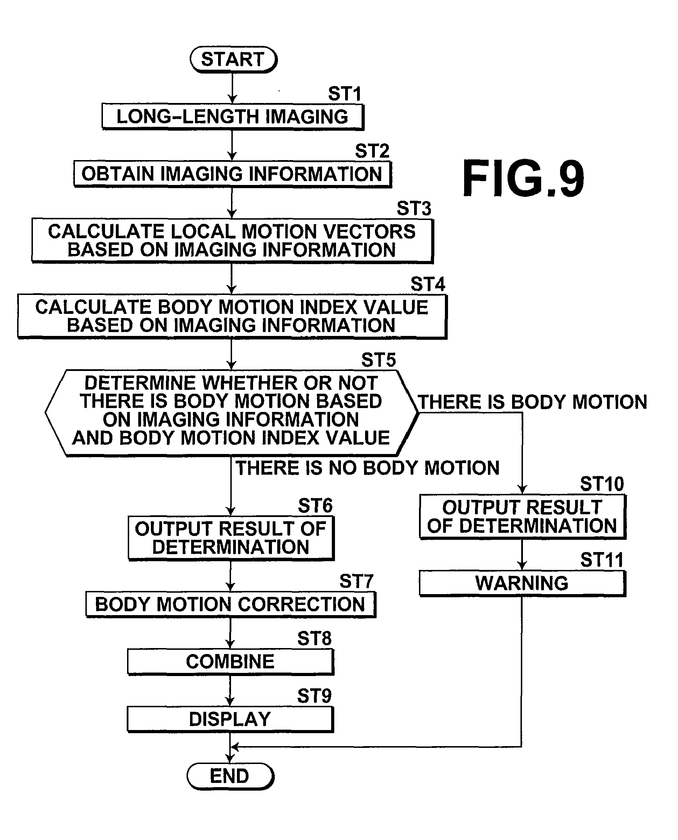

Next, a process carried out in the first embodiment is described. FIG. 9 is a flow chart illustrating the process carried out in the first embodiment. First, the long-length imaging is conducted with moving the FPD 110 to take a radiographic image at each position along the movement path (step ST1). Then, the imaging information obtaining unit 32 of the body motion detection unit 30 obtains the imaging information from the console 70 (step ST2). Subsequently, the local motion vector calculation unit 34 calculates the local motion vectors V0 in the overlapping area between each two adjacent radiographic images based on the imaging information (step ST3), and the body motion index value calculation unit 36 calculates the body motion index value based on the imaging information (step ST4). Then, the body motion determination unit 38 determines whether or not there is a body motion based on the imaging information and the body motion index value (step ST5).

If it is determined that there is no body motion, the body motion determination unit 38 outputs the result of determination together with the body motion index value to the body motion correction unit 40 and the image display unit 60 (step ST6). The body motion correction unit 40 corrects for a body motion in the radiographic images based on the body motion index value (step ST7), and the image combining unit 42 combines the radiographic images, which have been subjected to the body motion correction, to generate the combined image C1 (step ST8). Then, the image display unit 60 displays the combined image C1 together with the body motion index value (step ST9), and the process ends.

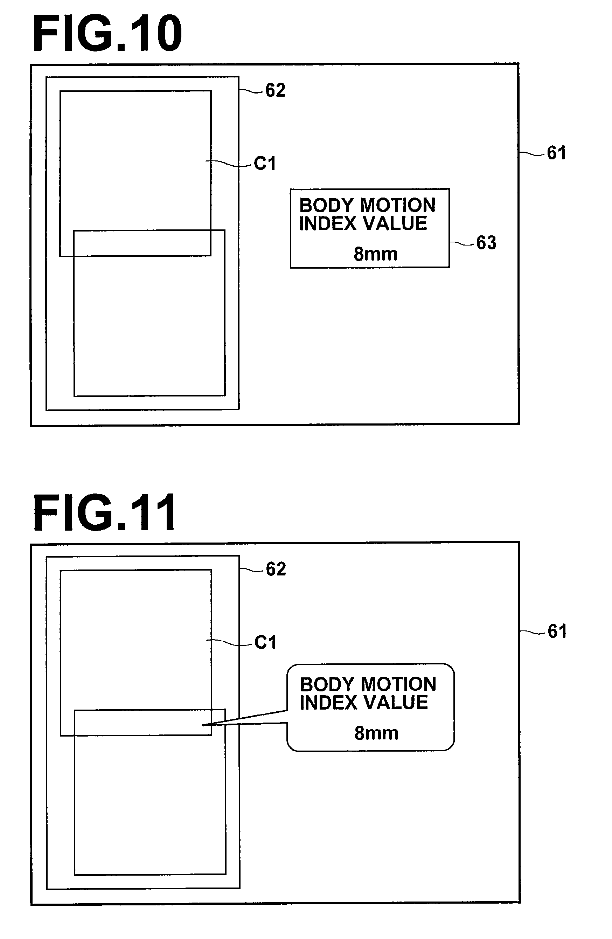

FIG. 10 is a diagram illustrating an example of display on a display screen of the image display unit 60 in the first embodiment. As shown in FIG. 10, the display screen 61 includes an image display area 62, where the combined image C1 is displayed, and a body motion index value display area 63, where the body motion index value is displayed. In the case where more than one types body motion index values are calculated, the body motion index values may be displayed on the body motion index value display area 63 with being associated with each of the parallel displacement, the three dimensional motion and the two dimensional motion. Alternatively, only the body motion index value of the parallel displacement may be displayed, or the largest body motion index value among the body motion index values of the parallel displacement, the three dimensional motion and the two dimensional motion may be displayed. In this case, since different units are used for the body motion index values of the parallel displacement, the three dimensional motion and the two dimensional motion, the units may be normalized to determine the magnitudes of the body motion index values. With respect to the body motion index value of the two dimensional motion, only one of the body motion index values of the rotation and the enlargement/reduction may be displayed, all the body motion index values may be displayed, or only the largest body motion index value maybe displayed. Alternatively, as shown in FIG. 11, the body motion index value may be displayed in popup when the cursor is moved near to the overlapping area of the combined image C1 displayed on the image display area 62.

In contrast, if it is determined in step ST5 that there is a body motion, the body motion determination unit 38 outputs the result of determination to the warning unit 44 (step ST10). The warning unit 44 makes a warning with a voice message (including a voice warning) or a buzzer sound (warning sound) (step ST11), and the process ends. In this case, the voice message may be one to prompt the operator to retake the images, such as "There is a large body motion. Please retake the images". Alternatively, a warning mark or a warning message may be displayed on the display screen of the image display unit 60. Still alternatively, the warning may be made with both the sound and the display. Yet alternatively, the result of determination and the body motion index value may be outputted to the image display unit 60, and only the body motion index value may be displayed on the image display unit 60 in the same manner as in the case where it is determined that there is no body motion.

As described above, in this embodiment, the body motion index value indicating the body motion of the subject during the radiographic imaging operations are obtained based on the imaging information representing the imaging conditions and the imaged subject during the imaging operations, and whether or not there is a body motion is determined based on the imaging information. Therefore, even when the imaging conditions and the imaged subject are changed, the body motion index value can be accurately obtained and whether or not there is a body motion can be accurately determined depending on the imaging conditions and the imaged subject, thereby achieving accurate detection of the body motion and accurate determination as to whether or not there is a body motion.

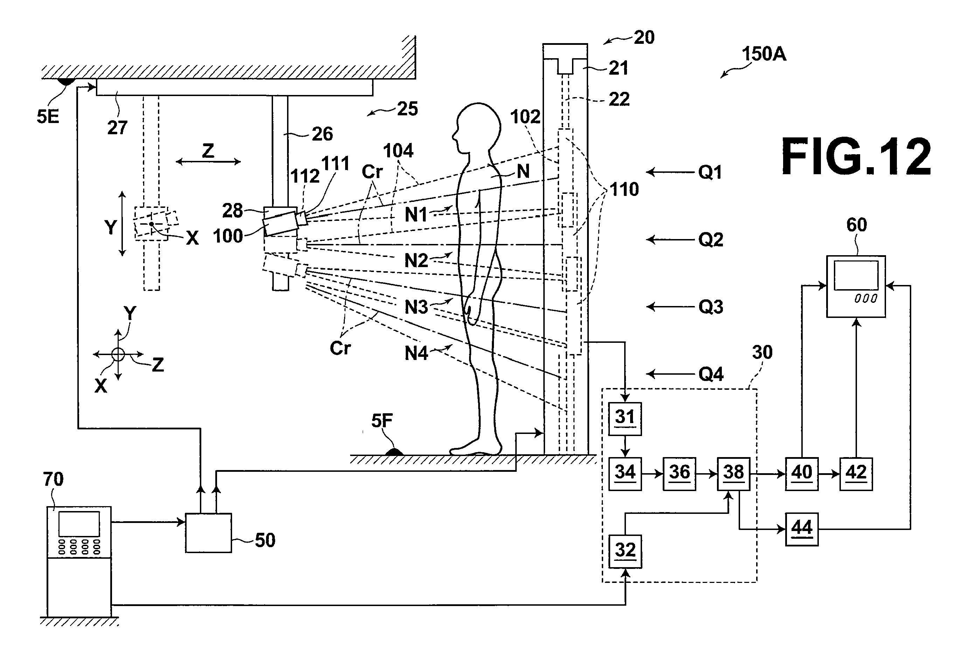

Next, a second embodiment of the invention is described. FIG. 12 is a schematic diagram illustrating the configuration of a radiographic imaging apparatus, to which a body motion detection device according to the second embodiment of the invention is applied. It should be noted that components in the second embodiment that are the same as those in the first embodiment are denoted by the same reference numerals and are not described in detail. The difference between a radiographic imaging apparatus 150A according to the second embodiment and the radiographic imaging apparatus of the first embodiment lies in that, in the second embodiment, only the body motion determination unit 38 of the body motion detection unit 30 determines whether or not there is a body motion based on the imaging information obtained by the imaging, information obtaining unit 32. That is, in the second embodiment, the local motion vector calculation unit 34 and the body motion index value calculation unit 36 calculate the local motion vectors V0 and the body motion index value according to a predetermined method without using the imaging information.

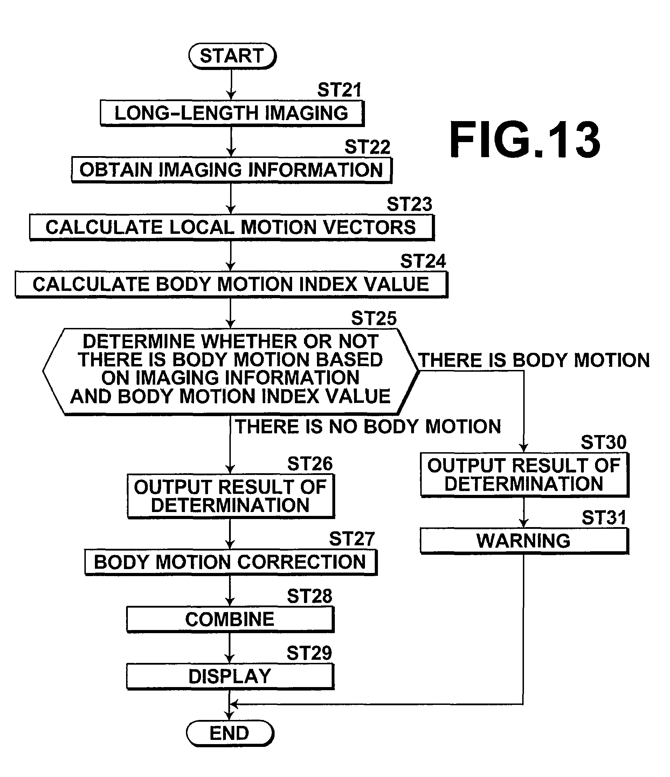

Next, a process carried out in the second embodiment of the invention is described. FIG. 13 is a flow chart illustrating the process carried out in the second embodiment. First, the long-length imaging is conducted with moving the FPD 110 to take a radiographic image at each position along the movement path (step ST21). Then, the imaging information obtaining unit 32 of the body motion detection unit 30 obtains the imaging information from the console 70 (step ST22). Subsequently, the local motion vector calculation unit 34 calculates the local motion vectors V0 in the overlapping area between each two adjacent radiographic images (step ST23), and the body motion index value calculation unit 36 calculates the body motion index value (step ST24). Then, the body motion determination unit 38 determines whether or not there is a body motion based on the imaging information and the body motion index value (step ST25).

If it is determined that there is no body motion, the body motion determination unit 38 outputs the result of determination together with the body motion index value to the body motion correction unit 40 and the image display unit 60 (step ST26). The body motion correction unit 40 corrects for a body motion in the radiographic images based on the body motion index value (step ST27), and the image combining unit 42 combines the radiographic images, which have been subjected to the body motion correction, to generate the combined image C1 (step ST28). Then, the image display unit 60 displays the combined image C1 together with the body motion index value (step ST29), and the process ends.

In contrast, if it is determined in step ST25 that there is a body motion, the body motion determination unit 38 outputs the result of determination to the warning unit 44 (step ST30). The warning unit 44 makes a warning with a voice message (including a voice warning) or a buzzer sound (warning sound) (step ST31), and the process ends.

It should be noted that, although the local motion vector calculation unit 34 calculates the local motion vectors V0 based on the imaging information and the body motion index value calculation unit 36 calculates the body motion index value based on the imaging information in the previously described first embodiment, only one of the local motion vector calculation unit 34 and the body motion index value calculation unit 36 may carry out the calculation based on the imaging information. Further, in the previously described first embodiment, the body motion determination unit 38 may determine whether or not there is a body motion without using the imaging information. In this case, the threshold value used for the determination may be fixed in advance.

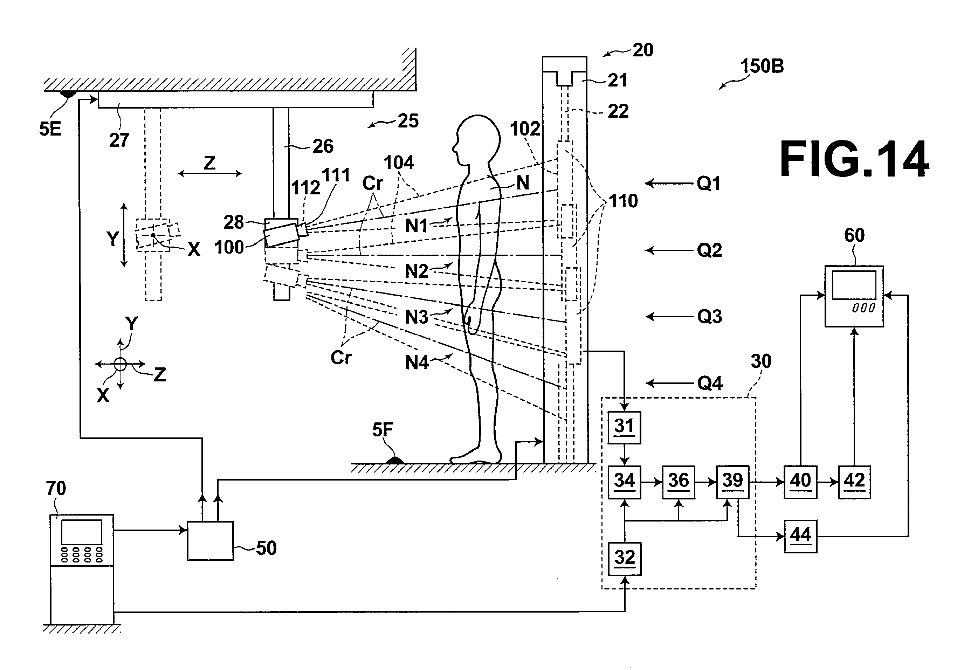

Next, a third embodiment of the invention is described. FIG. 14 is a schematic diagram illustrating the configuration of a radiographic imaging apparatus, to which a body motion detection device according to the third embodiment of the invention is applied. It should be noted that components in the third embodiment that are the same as those in the first embodiment are denoted by the same reference numerals and are not described in detail. The difference between a radiographic imaging apparatus 150B according to the third embodiment and the radiographic imaging apparatus of the first embodiment lies in that, in the third embodiment, a post-processing selection unit 39 is provided in the body motion detection unit 30 in place of the body motion determination unit 38.

It should be noted that, in the third embodiment, the local motion vector calculation unit 34 is not limited to calculate the local motion vectors V0 based on the imaging information as described above. Which of the above-described methods is used by the local motion vector calculation unit 34 to calculate the local motion vectors V0 may be determined in advance. In this case, the imaging information obtaining unit 32 is not necessary.

Further, in the third embodiment, the body motion index value calculation unit 36 is not limited to calculate all the body motion index values of the parallel displacement, the three dimensional motion and the two dimensional motion. The body motion index value calculation unit 36 may calculate at least two of the body motion index values of the parallel displacement, the three dimensional motion and the two dimensional motion. However, it is preferred that the body motion index value of the parallel displacement is included.

Further, in the third embodiment, the body motion index value calculation unit 36 is not limited to calculate the body motion index value based on the imaging information as described above. Which of the above-described methods is used by the body motion index value calculation unit 36 to calculate the body motion index value may be determined in advance. In this case, the imaging information obtaining unit 32 is not necessary.

The post-processing selection unit 39 selects processing to be carried out in a later stage based on the more than one types body motion index values depending on the motion of the subject, which are calculated as described above. To this end, the post-processing selection unit 39 first compares each body motion index value calculated by the body motion index value calculation unit 36 with a threshold value, and if the body motion index value is equal to or higher than the threshold value, it is determined that there is a body motion. In the third embodiment, the body motion index values of the parallel displacement, the three dimensional motion and the two dimensional motion are calculated, and each body motion index value is compared with a corresponding threshold value to determine whether or not there is a body motion for each body motion index value. With respect to the two dimensional motion, a threshold value for each of the rotation and the enlargement/reduction is prepared to determine whether or not there is a body motion for each of the rotation and the enlargement/reduction.

In the third embodiment, the post-processing selection unit 39 determines whether or not there is a body motions by comparing each body motion index value with the threshold value based on the imaging information calculated by the imaging information obtaining unit 32. Specifically, the magnitude of the threshold value is changed depending on the imaging information. For example, if the imaged body part is the leg (in the case other than the long-length imaging) or the entire leg (in the case of the long-length imaging), there is no structure that moves independently from the body motion of the subj ect N, such as the heart or gas in the intestine. In contrast, if the imaged body part is the chest (in the case other than the long-length imaging) or the entire spine (in the case of the long-length imaging), there is a structure that moves independently from the body motion of the subject N, such as the heart or gas in the intestine. Therefore, if the imaging information contains information indicating that the imaged body part is the chest, a larger threshold value than that used in the case where the imaged body part is the leg is used for the determination. In this manner, the determination as to whether or not there is a body motion can be achieved without being influenced by a local motion.

It should be noted that, in the third embodiment, the determination as to whether or not there is a body motion may be achieved with using threshold values fixed in advance, without based on the imaging information. In this case, the imaging information obtaining unit 32 is not necessary.

Further, the post-processing selection unit 39 selects whether or not the body motion correction is to be conducted by the body motion correction unit 40. Specifically, if it is determined that there is a body motion of the three dimensional motion, the body motion correction is not carried out regardless of whether or not there is a body motion of the parallel displacement and of the two dimensional motion. If it is determined that there is a body motion of the two dimensional motion, the body motion correction is not carried out regardless of whether or not there is a body motion of the parallel displacement and the three dimensional motion. With respect to the body motion of the parallel displacement, if it is determined that there is no body motion of the three dimensional motion and the two dimensional motion, the body motion correction is conducted regardless of whether or not there is a body motion. That is, the body motion correction is conducted in cases other than the case where it is determined that there is a body motion of any of the three dimensional motion and the two dimensional motion.

Further, the post-processing selection unit 39 selects the method used to achieve the body motion correction if it is selected that the body motion correction is to be conducted based on the result of determination as to whether or not there is a body motion and the body motion index values. It should be noted that, in this embodiment, each body motion index value is compared with the corresponding threshold value, and it is determined that there is no body motion if the body motion index value is less than the threshold value. Therefore, even when it is determined that there is no body motion, the body motion index value is not necessarily 0 and there may be a body motion. If there is a body motion of the three dimensional motion, nonlinear warping is selected. If there is a body motion of the rotation of the two dimensional motion, a method to achieve the body motion correction by rotation according to the above-described equation (2) is selected. If there is a body motion of the enlargement/reduction of the two dimensional motion, a method to achieve the body motion correction by enlargement or reduction according to the above-described equation (3) is selected. It should be noted that the method using nonlinear warping may be selected when there is a body motion of the two dimensional motion. If there is a body motion of both the three dimensional motion and the two dimensional motion, the method using nonlinear warping is selected. In cases other than the above-described cases, a method to achieve the body motion correction for the parallel displacement by parallel shifting based on the direction of the local motion vectors V0 is selected.

Further, the post-processing selection unit 39 selects the body motion index value to be displayed on the image display unit 60, which will be described later, based on the result of determination as to whether or not there is a body motion and the body motion index values. Specifically, if it is determined that there is a body motion of the three dimensional motion, or if there is a body motion of the three dimensional motion even when it is determined that there is no body motion of the three dimensional motion, it is selected to display the body motion index value of the three dimensional motion (i.e., the standard deviation or variance). If it is determined that there is a body motion of the rotation of the two dimensional motion, or if there is a body motion of the rotation even when it is determined that there is no body motion of the rotation, it is selected to display the body motion index value of the rotation (i.e., the rotational angle θ). If it is determined that there is a body motion of the enlargement/reduction of the two dimensional motion, or if there is a body motion of the enlargement/reduction even when it is determined that there is no body motion of the enlargement/reduction, it is selected to display the body motion index value of the enlargement/reduction (i.e., the parameters a and d). With respect to the body motion of the parallel displacement, it is selected to display the body motion index value of the parallel displacement.

In the third embodiment, if the post-processing selection unit 39 has selected that the body motion correction is to be conducted, the bodymotion correction unit 40 applies correction for eliminating an image distortion caused by a body motion of the subject N to the radiographic images with using the body motion correction method selected by the post-processing selection unit 39. In the case where the bodymotion correction for the parallel displacement is selected, the body motion index value of the parallel displacement has been detected, as shown at "a" in FIG. 8. Therefore, as shown at "b" in FIG. 8, the body motion correction is achieved by shifting the two radiographic images S1 and S2 relative to each other based on the body motion index value, i.e., the local motion vectors V0. In the case where the nonlinear warping is selected, the body motion correction is achieved by nonlinearly warping the radiographic images relative to each other. In the case where the body motion correction for the rotation or the enlargement/reduction is selected, the body motion correction is achieved according to the above-described equation (2) or (3).

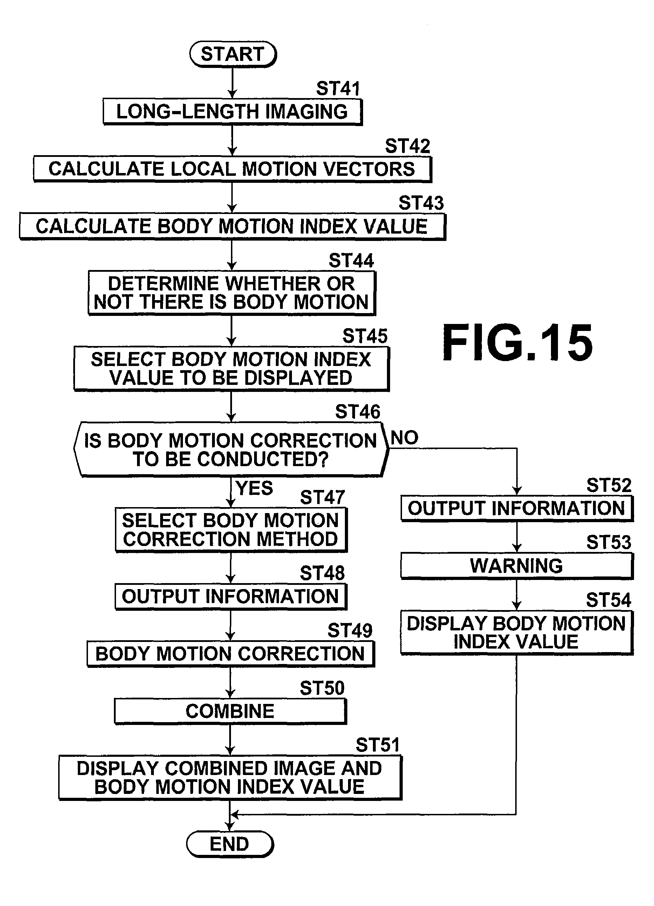

Next, a process carried out in the third embodiment is described. FIG. 15 is a flow chart illustrating the process carried out in the third embodiment. First, the long-length imaging is conducted with moving the FPD 110 to take a radiographic image at each position along the movement path (step ST41) . Then, the local motion vector calculation unit 34 calculates the local motion vectors V0 in the overlapping area between each two adjacent radiographic images (step ST42), and the body motion index value calculation unit 36 calculates the body motion index value (step ST43).

Subsequently, the post-processing selection unit 39 determines whether or not there is a body motion based on the body motion index values (step ST44) . Then, the post-processing selection unit 39 selects the body motion index value to be displayed on the image display unit 60 based on the result of determination as to whether or not there is a body motion and the body motion index values (step ST45), and selects whether or not the body motion correction is to be conducted depending on the result of determination as to whether or not there is a body motion (step ST46) . If an affirmative determination is made in step ST46, the post-processing selection unit 39 selects the method used to achieve the body motion correction (step ST47), and outputs the body motion index value used to achieve the body motion correction and information of the selected body motion correction method to the body motion correction unit 40, and outputs the body motion index value selected to be displayed to the image display unit 60, respectively (output information, step ST48).

Then, the body motion correction unit 40 corrects for the body motion in the radiographic images with using the body motion correction method selected by the post-processing selection unit 39 (step ST49), and the image combining unit 42 combines the radiographic images, which have been subjected to the body motion correction, to generate the combined image C1 (step ST50). Then, the image display unit 60 displays the combined image C1 together with the bodymotion index value selected to be displayed (step ST51), and the process ends.

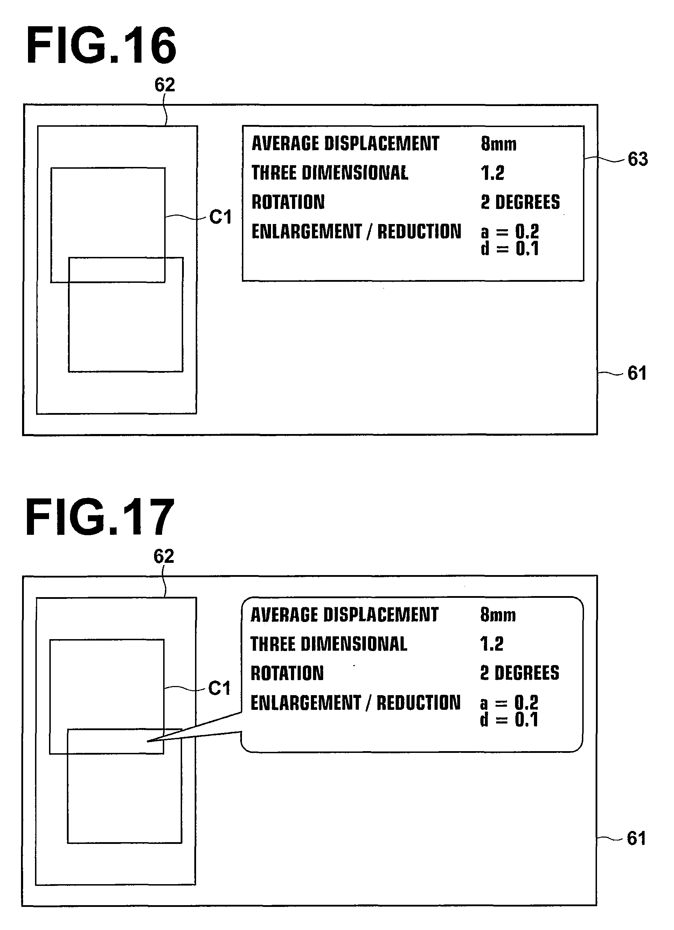

FIG. 16 is a diagram illustrating an example of display on the display screen of the image display unit 60 in the third embodiment. As shown in FIG. 16, the display screen 61 includes the image display area 62, where the combined image C1 is displayed, and the body motion index value display area 63, where the body motion index value is displayed. It should be noted that only the body motion index value which has been selected to be displayed is displayed on the body motion index value display area 63. FIG. 16 shows a state where the body motion index values of the parallel displacement, the three dimensional motion, and the rotation and the enlargement/reduction of the two dimensional motion are displayed. It should be noted that, as shown in FIG. 17, the body motion index values may be displayed in popup when the cursor is moved near to the overlapping area of the combined image C1 displayed on the image display area 62. In contrast, if a negative determination is made in step ST46, the post-processing selection unit 39 outputs the result of the selection indicating that the body motion correction is not to be conducted to the warning unit 44, and outputs the body motion index value selected to be displayed to the image display unit 60 (output information, step ST52). The warning unit 44 makes a warning with a voice message (including a voice warning) or a buzzer sound (warning sound) (step ST53). Further, the image display unit 60 displays the bodymotion index value which has been selected to be displayed (step ST54), and the process ends. In this case, the voice message may be one to prompt the operator to retake the images, such as "There is a large body motion. Please retake the images". Alternatively, a warning mark or a warning message may be displayed on the display screen of the image display unit 60. Still alternatively, the warning may be made with both the sound and the display.

As described above, in the third embodiment, more than one types of body motion index values are obtained depending on the body motion of the subject during the radiographic imaging operations. Therefore, appropriate body motion index values of the parallel displacement, the three dimensional motion and the two dimensional motion can be obtained depending on the body motion of the subject, thereby achieving accurate body motion detection. Further, the selection as to whether or not the body motion correction is to be conducted in a later stage, the selection of the body motion correction method to be used and the selection of the body motion index value to be displayed can appropriately be achieved based on the detected body motion index values.

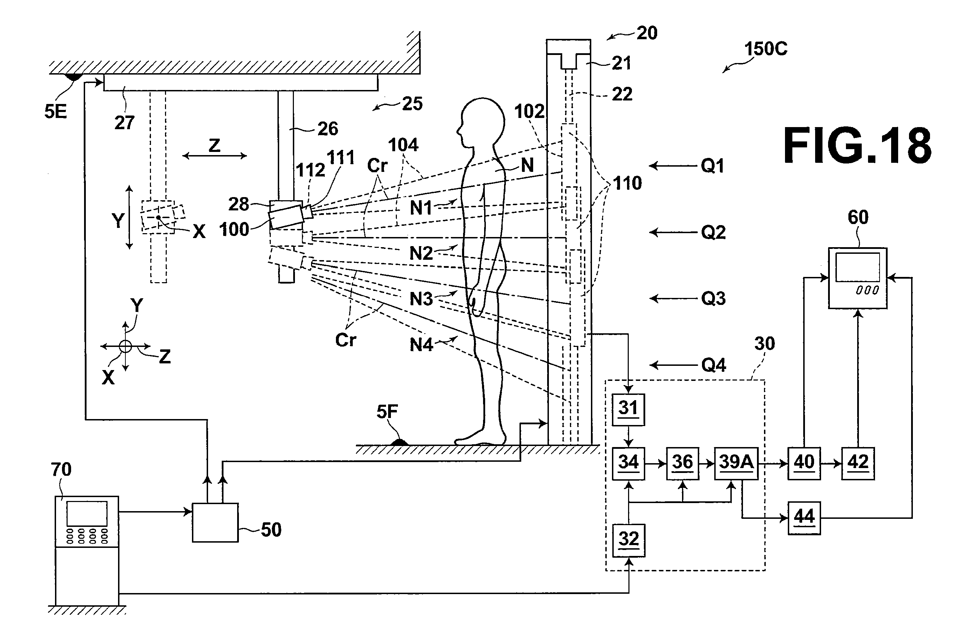

Next, a fourth embodiment of the invention is described. FIG. 18 is a schematic diagram illustrating the configuration of a radiographic imaging apparatus, to which a body motion detection device according to the fourth embodiment of the invention is applied. It should be noted that components in the fourth embodiment that are the same as those in the third embodiment are denoted by the same reference numerals and are not described in detail. The difference between a radiographic imaging apparatus 150C according to the fourth embodiment and the radiographic imaging apparatus of the third embodiment lies in that, in the fourth embodiment, the post-processing selection unit 39A determines the largest body motion index value among the body motion index values of the parallel displacement, the three dimensional motion and the two dimensional motion as a primary body motion index value, and the selection as to whether or not the body motion correction is to be conducted, the selection of the body motion correction method to be used and the selection of the body motion index value to be displayed are achieved based on the primary body motion index value.

That is, in the fourth embodiment, the post-processing selection unit 39A determines the magnitudes of the body motion index values of the parallel displacement, the three dimensional motion and the two dimensional motion calculated by the body motion index value calculation unit 36. In this case, since different units are used for the body motion index values of the parallel displacement, the three dimensional motion and the two dimensional motion, the units may be normalized to determine the magnitudes of the bodymotion index values. Then, the largest body motion index value among them is determined as the primary body motion index value, and whether or not the body motion correction is to be conducted is selected based on the primary body motion index value. Namely, if the primary body motion index value is the body motion index value of the three dimensional motion or the two dimensional motion, the body motion index value is compared with the threshold value to select whether or not the body motion correction is to be conducted. If the primary body motion index value is the body motion index value of the parallel displacement, it is selected that the body motion correction is to be conducted if it is determined that there is no body motion of the three dimensional motion and the two dimensional motion.

Further, if it is selected that the body motion correction is to be conducted, the method used to achieve the body motion correction is selected. Namely, if the primary body motion index value is the body motion index value of the three dimensional motion, the nonlinear warping is selected. If the primary body motion index value is the body motion index value of the rotation of the two dimensional motion, the method to achieve the body motion correction by rotation according to the above-described equation (2) is selected. If the primary body motion index value is the body motion index value of the enlargement/reduction of the two dimensional motion, the method to achieve the body motion correction by enlargement or reduction according to the above-described equation (3) is selected. It should be noted that the method using nonlinear warping may be selected when there is a body motion of the two dimensional motion. If the primary body motion index value is the body motion index value of the parallel displacement, the method to achieve the body motion correction for the parallel displacement by the parallel shifting based on the direction of the local motion vectors V0 is selected.

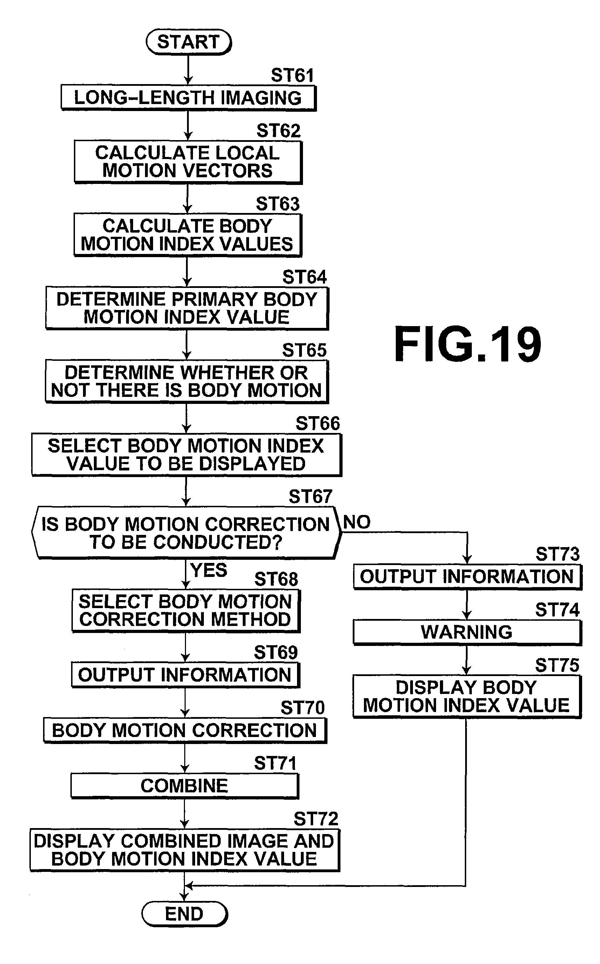

Next, a process carried out in the fourth embodiment of the invention is described. FIG. 19 is a flow chart illustrating the process carried out in the fourth embodiment. First, the long-length imaging is conducted with moving the FPD 110 to take a radiographic image at each position along the movement path (step ST61). Subsequently, the local motion vector calculation unit 34 calculates the local motion vectors V0 in the overlapping area between each two adjacent radiographic images (step ST62), and the body motion index value calculation unit 36 calculates the body motion index values (step ST63).

Subsequently, the post-processing selection unit 39 compares the magnitudes of the body motion index values with each other to determine the primary body motion index value (step ST64) . Further, the post-processing selection unit 39 determines whether or not there is a body motion based on the primary body motion index value (step ST65). Then, the post-processing selection unit 39 selects the primary body motion index value as the body motion index value to be displayed on the image display unit 60 (step ST66), and selects whether or not the body motion correction is to be conducted depending on the result of the determination as to whether or not there is a body motion with respect to the primary body motion index value (step ST67). If an affirmative determination is made in step ST67, the post-processing selection unit 39 selects the method used to achieve the body motion correction based on the primary body motion index value (step ST68), and outputs the primary body motion index value and information of the selected body motion correction method to the body motion correction unit 40 and outputs the primary body motion index value selected to be displayed to the image display unit 60, respectively (output information, step ST69).

Then, the body motion correction unit 40 corrects for the body motion in the radiographic images using the body motion correction method selected by the post-processing selection unit 39 (step ST70), and the image combining unit 42 combines the radiographic images, which have been subjected to the body motion correction, to generate the combined image C1 (step ST71). Then, the image display unit 60 displays the combined image C1 together with the body motion index value selected to be displayed (step ST72), and the process ends.

In contrast, if a negative determination is made in step ST67, the post-processing selection unit 39 outputs the result of the selection indicating that the body motion correction is not to be conducted to the warning unit 44, and outputs the primary body motion index value selected to be displayed to the image display unit 60 (output information, step ST73) . The warning unit 44 makes a warning with a voice message (including a voice warning) or a buzzer sound (warning sound) (step ST74). Further, the image display unit 60 displays the body motion index value which has been selected to be displayed (step ST75), and the process ends.

It should be noted that, in the previously-described third embodiment, the post-processing selection unit 39 may select more than one types of the body motion correction methods. For example, if there is a body motion including all the parallel displacement, the three dimensional motion and the two dimensional motion, all the body motion correction methods including the parallel shifting, the nonlinear warping, the rotation and the enlargement or reduction are selected. It should be noted that, with respect to the two dimensional motion, the nonlinear warping may sometimes be selected. Therefore, the body motion correction unit 40 may conduct the body motion correction with using all the possible combinations or any combination of the body motion correction methods selected by the post-processing selection unit 39. For example, if all the body motion correction methods including the parallel shifting, the nonlinear warping, the rotation and the enlargement or reduction are selected, there are the four types of body motion correction methods, and it is possible to conduct the body motion correction with using all the four types of body motion correction methods (there is a single combination), using three of the four types of body motion correction methods (there are four different combinations), using two of the four types of body motion correction methods (there are six different combinations), or using one of the four types of body motion correction methods (there are four different combinations or patterns). That is, the total of 15 different combinations or patterns of body motion correction methods are available to achieve the body motion correction.

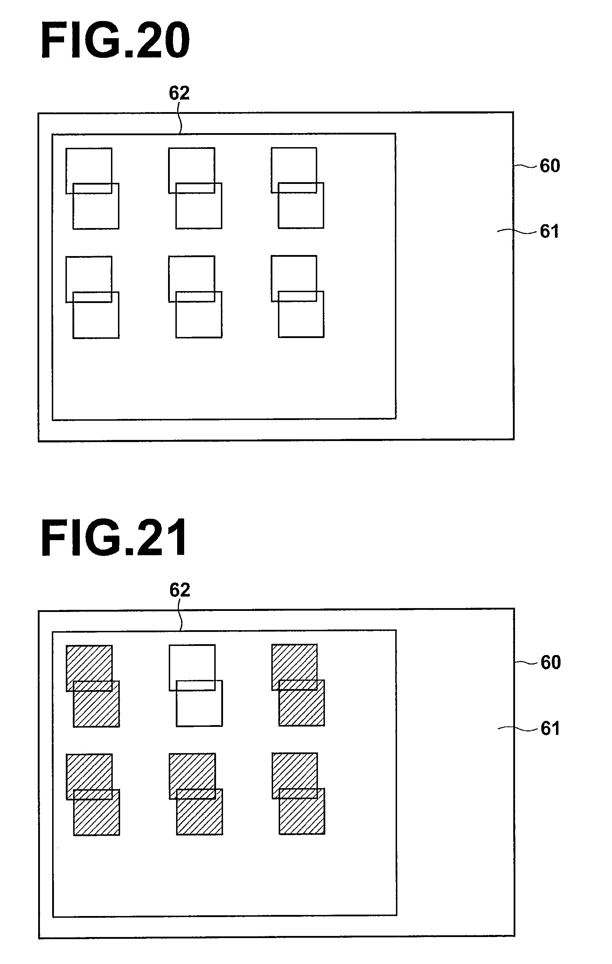

In this case, the body motion correction unit 40 may use all or any number of the 15 patterns of the body motion correction methods to achieve the body motion correction. Then, the image combining unit 42 generates a plurality of combined images with using all the radiographic images which have been corrected by the different patterns of body motion correction methods applied. Then, the image display unit 60 displays the plurality of combined images. FIG. 20 shows a state where the plurality of combined images are displayed. It should be noted that FIG. 20 shows the case where six patterns of the body motion correction methods are conducted and six combined images are displayed. As shown in FIG. 20, the image display area 62 of the display screen 61 contains reduced views of the combined images displayed in the form of a list. In this case, it may be preferred to display selected one of the combined images in an enlarged view. This allows the operator to select one of the combined images that has been corrected by a preferred pattern of the body motion correction methods. In this case, the combined images other than the combined image that has been corrected by the most preferred pattern of the body motion correction methods may be grayed out, as shown in FIG. 21. In FIG. 21, the grayed out state is indicated by the hatching.

It should be noted that, although the post-processing selection unit 39 selects whether or not the body motion correction is to be conducted, selects the body motion correction method to be used and selects the body motion index value to be displayed on the image display unit 60 in the above-described third and fourth embodiments, the post-processing selection unit 39 may select any one or any combination of these matters.

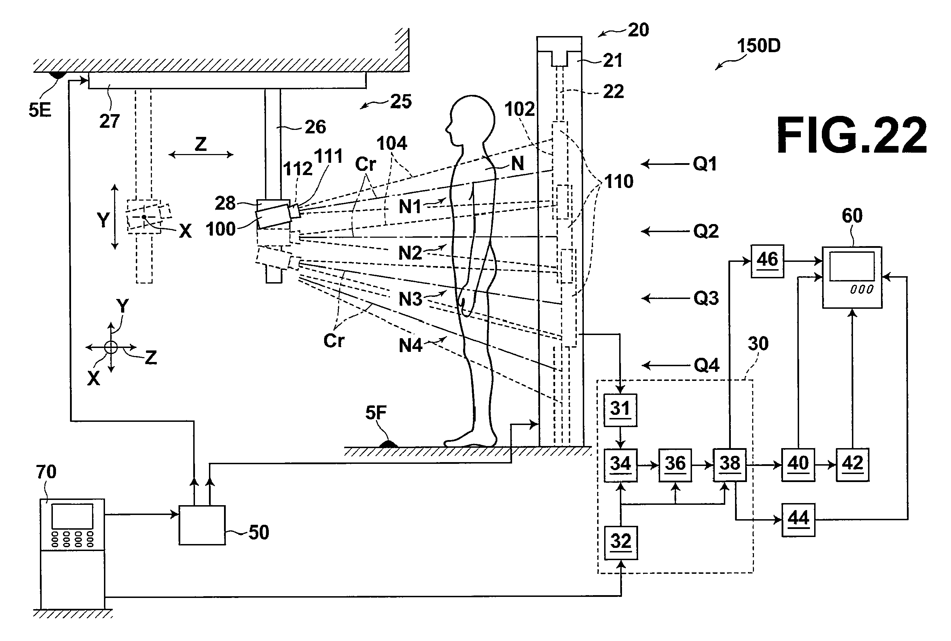

Next, a fifth embodiment of the invention is described. FIG. 22 is a schematic diagram illustrating the configuration of a radiographic imaging apparatus according to the fifth embodiment of the invention. It should be noted that components in the fifth embodiment that are the same as those in the first embodiment are denoted by the same reference numerals and are not described in detail. The difference between a radiographic imaging apparatus 150D according to the fifth embodiment and the radiographic imaging apparatus of the first embodiment lies in that a retake assisting information generating unit 46 is provided in the fifth embodiment.

The retake assisting information generating unit 46 generates retake assisting information for assisting retake of the image if it is determined by the body motion determination unit 38 that there is a body motion. For achieving the long-length imaging, it is necessary to retake the images if there is a large body motion between the imaging operations. Therefore, based on the result of determination by the body motion determination unit 38, the retake assisting information generating unit 46 generates, as the retake assisting information, information identifying two adjacent radiographic images with a body motion therebetween and information to be displayed on the image display unit 60 for conducting the retake with a reduced body motion. The information to be displayed on the image display unit 60 may include information, such as a text, an image (including a still image and a moving image) and a sound, to prompt the operator to secure the body part of the subject N where the body motion occurred. It should be noted that the information to be displayed when it is determined that there is a body motion may be one that recommends the operator to conduct a single shot imaging operation with using a single large FPD or CR long-length cassette, or the like, rather than the long-length imaging to take a plurality of radiographic images. The text and the image may be prepared in advance and stored in a storage unit (not shown).

Although the body motion index value calculation unit 36 calculates the body motion index value based on the imaging information, as described above, in the fifth embodiment, which of the above-described methods is used by the body motion index value calculation unit 36 to calculate the body motion index value may be determined in advance. In this case, the imaging information obtaining unit 32 is not necessary.

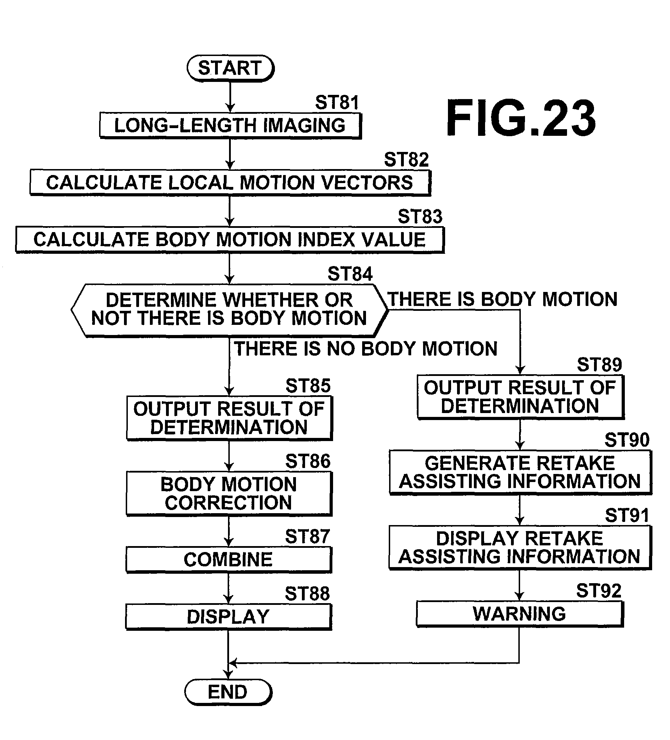

Next, a process carried out in the fifth embodiment is described. FIG. 23 is a flow chart illustrating the process carried out in the fifth embodiment. First, the long-length imaging is conducted with moving the FPD 110 to take a radiographic image at each position along the movement path (step ST81). Subsequently, the local motion vector calculation unit 34 calculates the local motion vectors V0 in the overlapping area between each two adjacent radiographic images (step ST82), and the body motion index value calculation unit 36 calculates the body motion index value (step ST83) . Then, the body motion determination unit 38 determines whether or not there is a body motion (step ST84).

If it is determined that there is no body motion, the body motion determination unit 38 outputs the result of determination together with the body motion index value to the body motion correction unit 40 and the image display unit 60 (step ST85). The body motion correction unit 40 corrects for a body motion in the radiographic images based on the body motion index value (step ST86), and the image combining unit 42 combines the radiographic images, which have been subj ected to the body motion correction, to generate the combined image C1 (step ST87). Then, the image display unit 60 displays the combined image C1 together with the body motion index value (step ST88), and the process ends.

The combined image C1 and the body motion index value are displayed on the image display unit 60 similarly to the state shown in FIG. 10 or 11 in the above-described first embodiment.

In contrast, if it is determined in step ST84 that there is a body motion, the body motion determination unit 38 outputs the result of determination to the warning unit 44 and the retake assisting information generating unit 46 (step ST89). The retake assisting information generating unit 46 generates the retake assisting information (step ST90), and outputs the retake assisting information to the image display unit 60. The image display unit 60 displays the retake assisting information (step ST91). On the other hand, the warning unit 44 makes a warning with a voice message (including a voice warning) or a buzzer sound (warning sound) (step ST92), and the process ends. In this case, the voice message may be one to prompt the operator to retake the images, such as "There is a large body motion. Please retake the images".

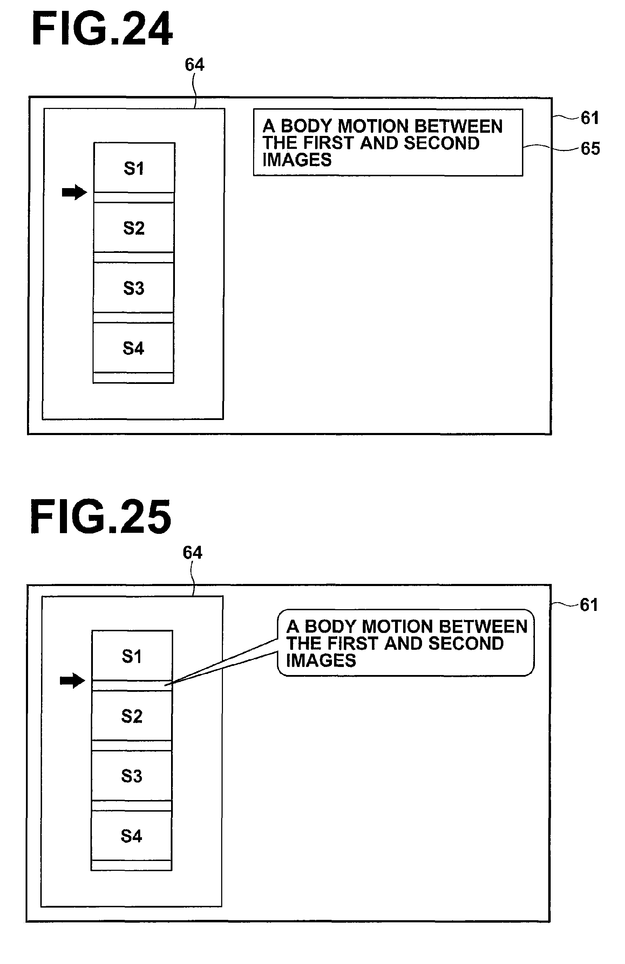

FIG. 24 is a diagram illustrating an example of display of the retake assisting information. As shown in FIG. 24, the image display area 64, which schematically shows the plurality of radiographic images (four radiographic images in this example) obtained by the long-length imaging, is displayed on the display screen 61 of the image display unit 60. As shown in FIG. 24, an arrow indicating a position where a body motion occurred is added as the retake assisting information to the four radiographic images S1 to S4 displayed on the display screen 61. The arrow shown in FIG. 24 indicates a position between the radiographic image S1 and the radiographic image S2, and it can be seen that a body motion occurred between the radiographic image S1 and the radiographic image S2. In place of the arrow, a symbol, etc., may be used. Further, as shown in FIG. 24, a text 65, "A body motion between the first and second images", is displayed as the retake assisting information. The operator viewing the retake assisting information can warn the subject N, secure the body part of the subject N corresponding to the position between the first and second images with a fastener, such as a belt, and/or secure the body part by placing a cushion, so that no body motion occurs between the first and second images during the retake operation.

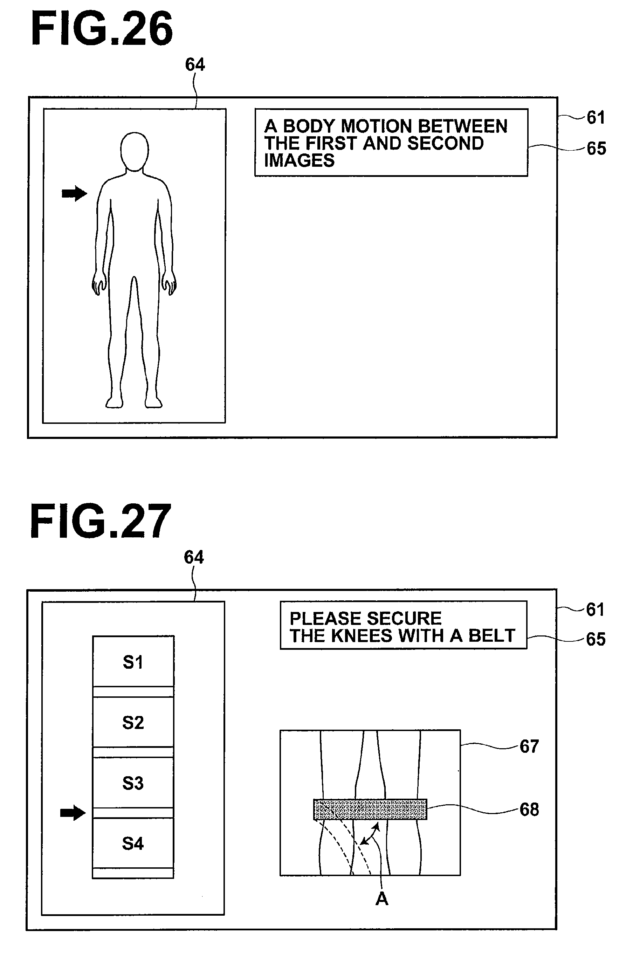

As shown in FIG. 25, the text may be displayed in popup when the cursor is moved near to a position where the arrow is added. Alternatively, as shown in FIG. 26, a schematic human body image 66 may be displayed on the image display area 64 in place of the four radiographic images S1 to S4, and the arrow may be provided at a position in the human body image 66 where the body motion occurred.

In place of or in addition to the text, a guidance image for prompting to secure the body part where a body motion occurred may be displayed. FIG. 27 shows a state where the guidance image is displayed. In FIG. 27, the guidance image is displayed together with the text. As shown in FIG. 27, an arrow indicating a position between the radiographic image S3 and the radiographic image S4 is provided on the image display area 64. The position between the radiographic image S3 and the radiographic image S4 corresponds to the position of the knee of the subject N. Therefore, the content of the text 65 is "Please secure the knees with a belt", and the guidance image 67 shows a state where the knees are secured with a belt, where the belt 68 in the guidance image 67 moving back and forth in the direction of arrow A is shown in animation.

It should be noted that, in the case where the retake assisting information recommends the operator to conduct a single shot imaging operation with using a single large FPD or CR long-length cassette, or the like, the content of the text may be "Please conduct a single shot imaging operation".

As described above, in the fifth embodiment, the retake assisting information is generated if it is determined that there is a body motion. Therefore, the operator can conduct the retake operation according to the retake instruction information so that no body motion occurs. This allows efficiently conducting the retake operation and reducing the exposure dose of the subject. In particular, in the case where the retake assisting information is displayed, the operator can check the retake assisting information at a glance, and thus can conduct the retake operation more efficiently.

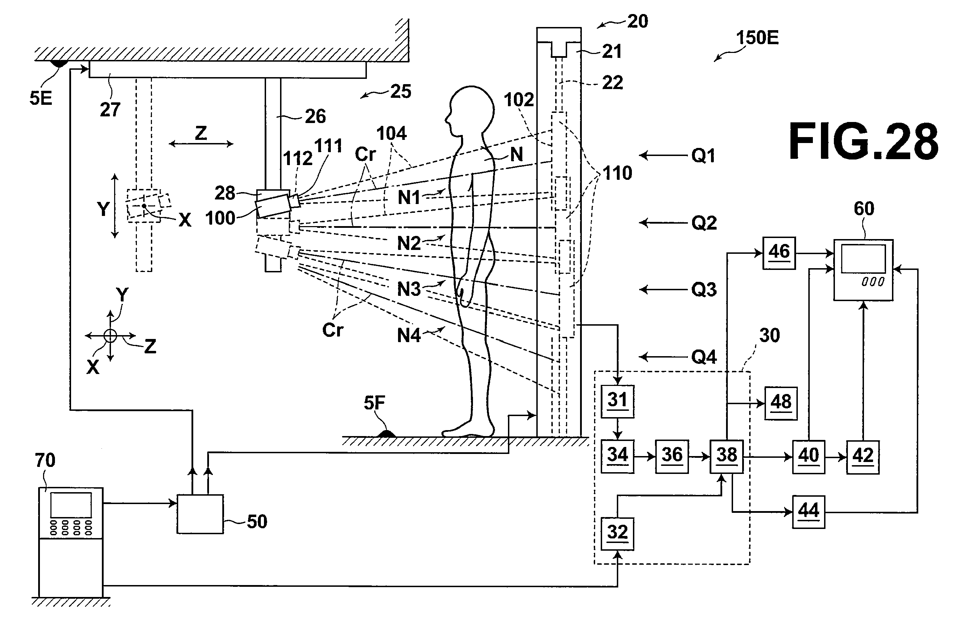

Next, a sixth embodiment of the invention is described. FIG. 28 is a schematic diagram illustrating the configuration of a radiographic imaging apparatus, to which a body motion detection device according to the sixth embodiment of the invention is applied. It should be noted that components in the sixth embodiment that are the same as those in the fifth embodiment are denoted by the same reference numerals and are not described in detail. The difference between a radiographic imaging apparatus 150E according to the sixth embodiment and the radiographic imaging apparatus of the fifth embodiment lies in that a retake control unit 48 is further provided in the sixth embodiment.

The retake control unit 48 instructs the above-described imaging adjusting unit to conduct the frame allocation again to avoid the position where a body motion occurred based on the retake assisting information generated by the retake assisting information generating unit 46. It should be noted that, in the case where the radiographic imaging apparatus 150E includes a fastener, such as a belt, for securing the subject N and the tightness of the fastener is adjustable, an instruction to tighten the fastener may be made. This instruction will hereinafter be referred to as "retake control".

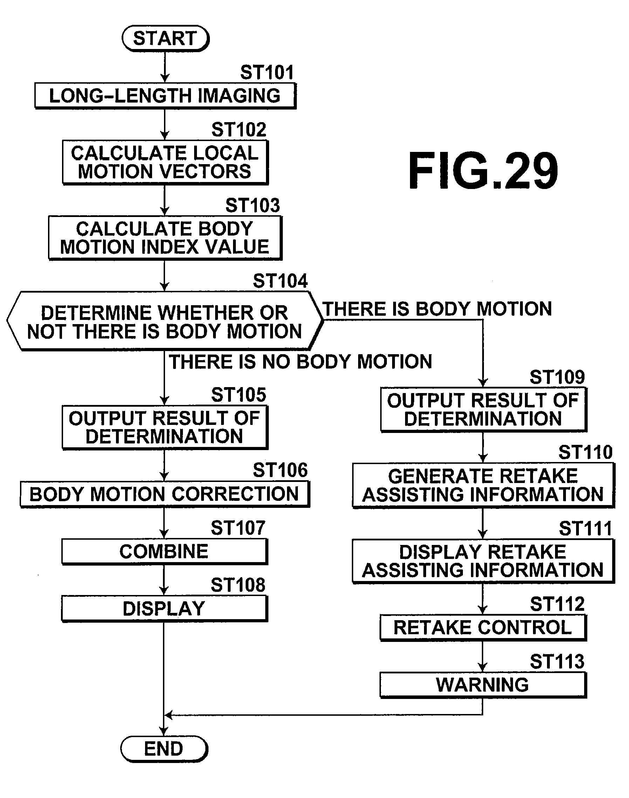

Next, a process carried out in the sixth embodiment of the invention is described. FIG. 29 is a flow chart illustrating the process carried out in the sixth embodiment. First, the long-length imaging is conducted with moving the FPD 110 to take a radiographic image at each position along the movement path (step ST101). Subsequently, the local motion vector calculation unit 34 calculates the local motion vectors V0 in the overlapping area between each two adjacent radiographic images (step ST102), and the body motion index value calculation unit 36 calculates the body motion index value (step ST103). Then, the body motion determination unit 38 determines whether or not there is a body motion (step ST104).

If it is determined that there is no body motion, the body motion determination unit 38 outputs the result of determination together with the body motion index value to the body motion correction unit 40 and the image display unit 60 (step ST105) . The body motion correction unit 40 corrects for a body motion in the radiographic images based on the body motion index value (step ST106), and the image combining unit 42 combines the radiographic images, which have been subjected to the body motion correction, to generate the combined image C1 (step ST107). Then, the image display unit 60 displays the combined image C1 together with the body motion index value (step ST108), and the process ends.

In contrast, if it is determined in step ST104 that there is a body motion, the body motion determination unit 38 outputs the result of determination to the warning unit 44 and the retake assisting information generating unit 46 (step ST109). The retake assisting information generating unit 46 generates the retake assisting information (step ST110), and outputs the retake assisting information to the image display unit 60 and the retake control unit 48. The image display unit 60 displays the retake assisting information (step ST111) . Further, the retake control unit 48 conducts the above-described retake control (step ST112). On the other hand, the warning unit 44 makes a warning with a voice message (including a voice warning) or a buzzer sound (warning sound) (step ST113), and the process ends.

It should be noted that, although the retake assisting information is displayed on the image display unit 60 in the above-described sixth embodiment, only the retake control may be conducted without displaying the retake assisting information.

Although the body motion index values of the parallel displacement, the three dimensional movement and the two dimensional movement are calculated in the first to sixth embodiments, it is known that the body motions of the subject during, in particular, the long-length imaging are mainly movements of the subject in the longitudinal and transverse directions relative to the FPD 110, i.e., the parallel displacements. The body motion of the parallel displacement can be corrected for by shifting the radiographic images relative to each other, and this does not degrade the image quality of the radiographic images. Therefore, when it is known that the body motion of the parallel displacement is within a range where it does not influence the measurement and diagnosis, no influence is exerted on image interpretation of the combined image, which is obtained by combining the radiographic images, by correcting for the body motion of the parallel displacement. If it can be known during the long-length imaging that the body motion is the parallel displacement and is within a range where it does not influence the measurement and diagnosis, it is not necessary to retake the radiographic images since correction of the radiographic images exerts no influence on image interpretation of the combined image, and as a result, needless radiation exposure of the subject can be prevented.

For this reason, when the body motion index value is calculated, only the body motion index value of the parallel displacement may be calculated. Now, this aspect is described as a seventh embodiment. The difference between the seventh embodiment and the above-described embodiments lies only in the operation conducted by the body motion index value calculation unit 36, and therefore the configuration of the seventh embodiment is not described in detail.

In the seventh embodiment, the body motion index value calculation unit 36 calculates only the body motion index value of the parallel displacement. Specifically, the histogram as shown in FIG. 6 is generated with using the local motion vectors V0 calculated by the local motion vector calculation unit 34, and the body motion index value of the parallel displacement is calculated based on the histogram. If the imaged body part is the chest (in the case other than the long-length imaging) or the entire spine (in the case of the long-length imaging), there is a structure that moves independently from the body motion of the subject N, such as the heart or gas in the intestine. That is, a local motion other than a motion relating to the posture of the subject N when the subject N is regarded as a rigid body is included. The body motion index value calculation unit 34 calculates the body motion index value with using the maximum frequency position or the frequency centroid position in the generated histogram. It should be noted that a weighted average of the histogram, where a larger weight is assigned to the local motion vectors with a larger frequency, maybe calculated as the body motion index value.

In this manner, only the bodymotion index value of the parallel displacement, which is a motion relating to the posture of the subj ect N when the subject N is regarded as a rigid body, can be calculated without being influenced by a local motion.

The method used to calculate the body motion index value of the parallel displacement is not limited to one that uses the histogram. For example, the body motion index value of the parallel displacement may be calculated with minimizing the influence of the local motion by a method which involves clustering the local motion vectors V0 into groups of the local motion vectors of the same direction and magnitude to separate the local motion vectors V0, and removing the local motion vector V0 with a magnitude less than a predetermined threshold value from the separated local motion vectors V0 or assigning a larger weight to the local motion vector V0 having a larger magnitude.

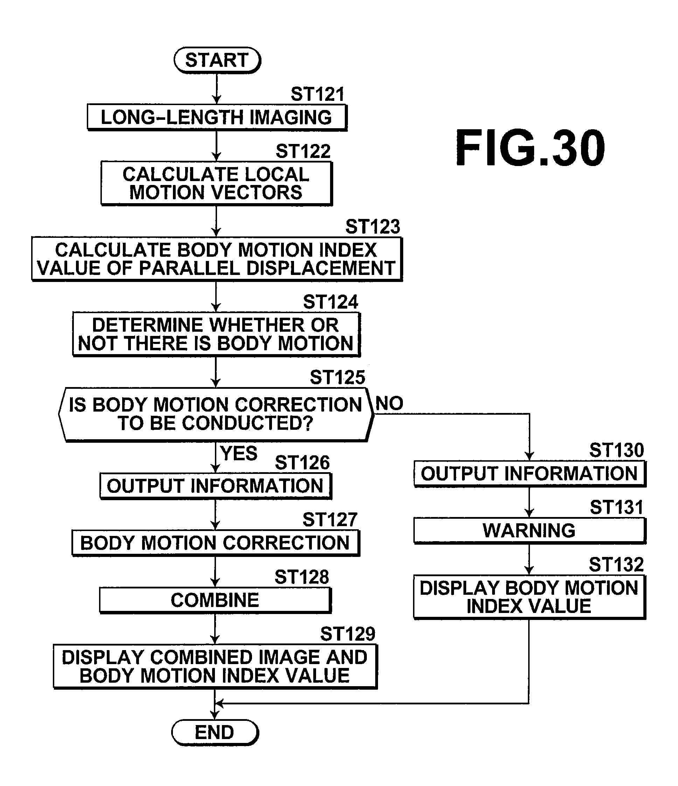

Next, a process carried out in the seventh embodiment is described. FIG. 30 is a flow chart illustrating the process carried out in the seventh embodiment. First, the long-length imaging is conducted with moving the FPD 110 to take a radiographic image at each position along the movement path (step ST121) . Then, the local motion vector calculation unit 34 calculates the local motion vectors V0 in the overlapping area between each two adjacent radiographic images (step ST122), and the body motion index value calculation unit 36 calculates the body motion index value of the parallel displacement (step ST123).

Subsequently, the post-processing selection unit 39 determines whether or not there is a body motion based on the body motion index value (step ST124) . Then, the post-processing selection unit 39 selects whether or not the body motion correction is to be conducted depending on the result of the determination as to whether or not there is a body motion (step ST125). If an affirmative determination is made in step ST125, the post-processing selection unit 39 outputs information of the body motion index value of the parallel displacement to the body motion correction unit 40 and to the image display unit 60, respectively (output information, step ST126) .

Then, the body motion correction unit 40 corrects for the body motion of the parallel displacement in the radiographic images (step ST127), and the image combining unit 42 combines the radiographic images, which have been subjected to the body motion correction, to generate the combined image C1 (step ST128). Then, the image display unit 60 displays the combined image C1 together with the body motion index value of the parallel displacement (step ST129), and the process ends.

In contrast, if a negative determination is made in step ST125, the post-processing selection unit 39 outputs the result of the selection indicating that the body motion correction is not to be conducted to the warning unit 44, and outputs the body motion index value of the parallel displacement to the image display unit 60 (output information, step ST130) . The warning unit 44 makes a warning with a voice message (including a voice warning) or a buzzer sound (warning sound) (step ST131). Further, the image display unit 60 displays the body motion index value of the parallel displacement (step ST132), and the process ends.

In this manner, in the seventh embodiment, accurate detection of the body motion of the parallel displacement of the subject can be achieved. Further, selection of whether or not the body motion correction is to be conducted in a later stage can appropriately be achieved based on the detected body motion index value of the parallel displacement.

Although the body motion index value with respect to radiographic images taken by the long-length imaging is obtained in the above-described first to fourth and seventh embodiments, the body motion detection unit 30 may be used alone to calculate the local motion vectors V0 and the body motion index value and determine whether or not there is a body motion based on the imaging information with respect to radiographic images taken by imaging operations of the same subject where a body motion of the subject may possibly occur between shots, such as energy subtraction imaging, temporal subtraction imaging, tomosynthesis imaging and continuous shooting, in the similar manner to the above-described third and fourth embodiments.

Further, in the above-described first to fourth and seventh embodiments, the calculation of the body motion index value and the determination as to whether or not there is a body motion may be conducted based on the overlapping area between each two adjacent radiographic images for each of the second and the following positions, i.e., for each of the second and the following imaging operations, while the FPD 110 is moved, i.e., during imaging, and if it is determined that there is a body motion, a warning may be made. Still further, in the above-described fifth and sixth embodiments, a warning may be made and the retake assisting information may be generated if it is determined that there is a body motion.

In this case, when the operator has recognized a body motion of the subject N during the long-length imaging in response to a warning, or the like, the operator can stop the imaging operation by operating an emergency stop switch provided at the console 70. This prevents needless radiation exposure of the subject N caused by continuing the imaging operation even when the there is a too large body motion to combine the taken images. Further, in the above-described fifth and sixth embodiments, the retake assisting information helps the operator to take a measure to prevent a body motion during the retake operation.

Although a warning is made if it is determined that there is a body motion of the subject during the long-length imaging in the above-described embodiments, the imaging operation may automatically be stopped when the warning is made. This eliminates the need of immediate operation of the emergency stop switch by the operator when the operator has recognized the body motion of the subject in response to a warning, or the like, or this prevents such a situation that the next imaging operation is conducted until the emergency stop switch is operated, thereby preventing needless radiation exposure of the subject without imposing a burden on the operator.

Further, although the local motion vector calculation unit 34 and the body motion index value calculation unit 36 detect the body motion index value in the above-described fifth and sixth embodiments, a sensor may be provided at the detector moving unit 20 to detect a body motion. The sensor may, for example, be a sensor disposed at a grab bar (not shown) for detecting rocking and twisting of the body of the subject, a sensor having weight scales disposed at positions where the right and left feet of the subject are put for detecting shifting of the body weight, or a position sensor disposed on the subject N.

The apparatus according to the embodiments of the invention has been described. The present invention may also be implemented in the form of a program for causing a computer to function as means corresponding to the imaging information obtaining unit 32, the local motion vector calculation unit 34, the body motion index value calculation unit 36, the body motion determination unit 38, the post-processing selection unit 39, the retake assisting information generating unit 46 and the retake control unit 48 described above to carry out the operation shown in FIG. 9, 13, 15, 19, 23, 29 or 30. Further, the present invention may also be implemented in the form of a computer readable recording medium containing the program.

Embodiment claims of the present invention are described below.

[Embodiment claim 1]

A body motion detection device comprising:

image obtaining means for obtaining a plurality of radiographic images taken by carrying out a plurality of imaging operations with respect to an identical subject, the radiographic images at least partially overlapping with one another; local motion vector calculating means for calculating at least one local motion vector representing a local displacement of the subject in an overlapping area between the radiographic images; and body motion index value calculating means for calculating a body motion index value attributed to a posture of the subject based on the local motion vector.

The "body motion index value attributed to a posture of the subject" refers to an index value of a body motion attributed to a posture of the subject when the subject is regarded as a rigid body, and does not include an index value attributed to a motion of an internal organ, such as the heart, lung, intestine, or the like, inside the subject.

[Embodiment claim 2]

The body motion detection device as defined in embodiment claim 1, wherein the body motion index value calculating means calculates an index value of an amount of parallel displacement of the subject as the body motion index value.

[Embodiment claim 3]

The body motion detection device as defined in embodiment claim 2, wherein the body motion index value calculating means further calculates at least one of an index value of an amount of three dimensional movement of the subject and an index value of an amount of two dimensional movement of the subject as the body motion index value.

[Embodiment claim 4]

The body motion detection device as defined in embodiment claim 3, further comprising post-processing selecting means for selecting post-processing with respect to the radiographic images based on the body motion index value.

[Embodiment claim 5]

The body motion detection device as defined in embodiment claim 4, wherein the post-processing selecting means selects whether or not to apply body motion correction to the radiographic images.

[Embodiment claim 6]

The body motion detection device as defined in embodiment claim 4 or 5, wherein the post-processing selecting means selects a type of body motion correction to be applied to the radiographic images.

[Embodiment claim 7]

The body motion detection device as defined in any one of embodiment claims 4 to 6, wherein the post-processing selecting means selects a type of body motion index value to be displayed.

[Embodiment claim 8]

The body motion detection device as defined in any one of embodiment claims 1 to 7, further comprising imaging information obtaining means for obtaining imaging information representing imaging conditions and an imaged subject during the imaging operations.

[Embodiment claim 9]

The body motion detection device as defined in embodiment claim 8, wherein the local motion vector calculating means calculates the at least one local motion vector based on the imaging information.

[Embodiment claim 10]

The body motion detection device as defined in embodiment claim 8 or 9, wherein the body motion index value calculating means calculates the body motion index value of the parallel displacement by integrating the at least one local motion vector based on the imaging information.

[Embodiment claim 11]

The body motion detection device as defined in any one of embodiment claims 8 to 10, further comprising body motion determining means for determining whether or not there is a body motion of the subject based on the imaging information and the body motion index value.

[Embodiment claim 12]

The body motion detection device as defined in any one of embodiment claims 1 to 10, further comprising body motion determining means for determining whether or not there is a body motion of the subject based on the body motion index value.

[Embodiment claim 13]

A radiographic imaging apparatus for obtaining a plurality of radiographic images at least partially overlapping with one another by shifting a position of a radiation detector and applying radiation transmitted through a subject to the radiation detector each time the position is shifted, the apparatus comprising:

imaging means for shifting the position of the radiation detector along a predetermined axis of movement, and applying radiation transmitted through the subject to the radiation detector each time the position is shifted;
radiographic image obtaining means for obtaining a plurality of radiographic images of the subject by reading out a signal from the radiation detector each time the position is shifted and the radiation is applied; and
the body motion detection device as defined in any one of embodiment claims 1 to 12.

[Embodiment claim 14]

The radiographic imaging apparatus as defined in embodiment claim 13, further comprising information generating means for generating retake assisting information for assisting retake of the radiographic images if the body motion is detected.

[Embodiment claim 15]

The radiographic imaging apparatus as defined in embodiment claim 14, further comprising display means for displaying the retake assisting information.

[Embodiment claim 16]

The radiographic imaging apparatus as defined in embodiment claim 14 or 15, further comprising retake controlling means for controlling the retake based on the retake assisting information.

[Embodiment claim 17]

A body motion detection method comprising:

obtaining a plurality of radiographic images taken by carrying out a plurality of imaging operations with respect to an identical subject, the radiographic images at least partially overlapping with one another;
calculating at least one local motion vector representing a local displacement of the subject in an overlapping area between the radiographic images; and
calculating a body motion index value attributed to a posture of the subject based on the local motion vector.

[Embodiment claim 18]

A program for causing a computer to carry out a body motion detection method comprising the steps of:

obtaining a plurality of radiographic images taken by carrying out a plurality of imaging operations with respect to an identical subject, the radiographic images at least partially overlapping with one another;
calculating at least one local motion vector representing a local displacement of the subject in an overlapping area between the radiographic images; and
calculating a body motion index value attributed to a posture of the subject based on the local motion vector.

**Claims**

1. A body motion detection device comprising:

   image obtaining means (31) for obtaining a plurality of radiographic images taken by carrying out a plurality of imaging operations with respect to an identical subject, the radiographic images at least partially overlapping with one another;
   imaging information obtaining means (32) for obtaining imaging information representing imaging conditions and an imaged subject during the imaging operations; and
   body motion index value obtaining means (34, 36, 38) for obtaining a body motion index value based on the imaging information, the body motion index value indicating a body motion of the subject during imaging of the radiographic images.

2. The body motion detection device as claimed in claim 1, wherein the body motion index value obtaining means comprises:

   local motion vector calculating means (34) for calculating at least one local motion vector representing a local displacement of the subject in an overlapping area between the radiographic images; and
   body motion index value calculating means (36) for calculating the body motion index value based on the local motion vector.

**3.** The body motion detection device as claimed in claim 2, wherein the body motion index value calculatingmeans (36) calculates an index value of an amount of parallel displacement of the subject as the body motion index value.

**4.** The body motion detection device as claimed in claim 3, wherein the body motion index value calculating means (36) further calculates at least one of an index value of an amount of three dimensional movement of the subject and an index value of an amount of two dimensional movement of the subject as the body motion index value.

**5.** The body motion detection device as claimed in any one of claims 1 to 4, further comprising body motion determining means (38) for determining whether or not there is a body motion of the subject based on the imaging information and the body motion index value.

**6.** The body motion detection device as claimed in any one of claims 1 to 5, wherein the imaging information comprises at least one of an imaging time during the imaging operations, an imaging interval between the radiographic images, whether or not the subject is immobilized during the imaging operations, an imaged body part of the subject and symptoms of the subject.

**7.** A body motion detection device comprising:

image obtaining means (31) for obtaining a plurality of radiographic images taken by carrying out a plurality of imaging operations with respect to an identical subject, the radiographic images at least partially overlapping with one another; and
body motion index value obtaining means (34, 36, 38) for obtaining different types of body motion index values according to types of motion of the subject during imaging of the radiographic images.

**8.** The body motion detection device as claimed in claim 7, wherein the body motion index value obtaining means comprises:

local motion vector calculating means (34) for calculating at least one local motion vector representing a local displacement of the subject in an overlapping area between the radiographic images; and
body motion index value calculating means (36) for calculating the different types of body motion index values based on the local motion vector.

**9.** The body motion detection device as claimed in claim 8, wherein the body motion index value calculating means (36) calculates at least two of an index value of an amount of parallel displacement of the subject, an index value of an amount of three dimensional movement of the subject and an index value of an amount of two dimensional movement of the subject as the body motion index values.

**10.** The body motion detection device as claimed in any one of claims 7 to 9, further comprising post-processing selecting means (39) for selecting post-processing with respect to the radiographic images based on the different types of body motion index values or a primary body motion index value among the different types of body motion index values.

**11.** The body motion detection device as claimed in claim 10, wherein the post-processing selecting means (39) determines a type of the motion of the subject for which a body motion is detected based on the different types of bodymotion index values or the primary body motion index value, and selects the post-processing depending on a result of the determination.

**12.** The body motion detection device as claimed in claim 10 or 11, wherein the post-processing selecting means (39) selects whether or not to apply body motion correction to the radiographic images.

**13.** The body motion detection device as claimed in any one of claims 10 to 12, wherein the post-processing selecting means (39) selects a type of body motion correction to be applied to the radiographic images.

**14.** The body motion detection device as claimed in any one of claims 10 to 13, wherein the post-processing selecting means (39) selects a type of body motion index value to be displayed.

**15.** A radiographic imaging apparatus for obtaining a plurality of radiographic images at least partially overlapping with one another by shifting a position of a radiation detector (110) and applying radiation transmitted through a subject to the radiation detector (110) each time the position is shifted, the apparatus comprising:

imaging means (20, 25, 100) for shifting the position of the radiation detector (110) along a predetermined axis of movement, and applying radiation transmitted through the subject to the radiation detector (110) each time the position is shifted;

radiographic image obtaining means (50) for obtaining a plurality of radiographic images of the subject by reading out a signal from the radiation detector (110) each time the position is shifted and the radiation is applied;

body motion detecting means (30) for detecting a body motion of the subject during imaging of the radiographic images; and

information generating means (46) for generating retake assisting information for assisting retake of the radiographic images if the body motion is detected.

16. The radiographic imaging apparatus as claimed in claim 15, further comprising display means (60) for displaying the retake assisting information.

17. The radiographic imaging apparatus as claimed in claim 15 or 16, further comprising retake controlling means (48) for controlling the retake based on the retake assisting information.

18. The radiographic imaging apparatus as claimed in claim 17, wherein the retake controlling means (48) carries out frame allocation for taking the radiographic images with avoiding a position of the subject where the body motion occurred.

19. The radiographic imaging apparatus as claimed in claim 17 or 18, wherein the retake controlling means (48) increases tightness of a fastener securing the subject.

20. The radiographic imaging apparatus as claimed in any one of claims 15 to 19, wherein the body motion detecting means (30) comprises:

local motion vector calculating means (34) for calculating at least one local motion vector representing a local displacement of the subject in an overlapping area between the radiographic images;

body motion index value calculatingmeans (36) for calculating the body motion index value based on the local motion vector; and

body motion determining means (38) for determining whether or not there is the body motion based on the body motion index value.

21. The radiographic imaging apparatus as claimed in claim 20, wherein the body motion index value calculating means (36) calculates an index value of an amount of parallel displacement of the subject as the body motion index value.

22. The radiographic imaging apparatus as claimed in claim 21, wherein the body motion index value calculating means (36) further calculates at least one of an index value of an amount of three dimensional movement of the subject and an index value of an amount of two dimensional movement of the subject as the body motion index value.

23. The radiographic imaging apparatus as claimed in any one of claims 15 to 19, wherein the body motion detecting means (30) comprises a sensor for detecting a motion of the subject.

**FIG.1**

EP 2 428 164 A2

FIG.2

# FIG.3

K1-1

K2-1

K1-2

K2-2

K1-3

K2-3

# FIG.4

I

R

# FIG.5

S1

T

K1

R    K2

I

S2

# FIG.6

FREQUENCY

HORIZONTAL
DISPLACEMENT

VERTICAL DISPLACEMENT

# FIG.7

FREQUENCY

STANDARD DEVIATION
OR VARIANCE

HORIZONTAL
DISPLACEMENT

VERTICAL DISPLACEMENT

# FIG.8A

S1

S2

# FIG.8B

S1

S2

**FIG.9**

```
                    ( START )
                        │
                        ▼           ST1
        ┌──────────────────────────────┐
        │     LONG-LENGTH IMAGING       │
        └──────────────────────────────┘
                        │           ST2
        ┌──────────────────────────────┐
        │   OBTAIN IMAGING INFORMATION  │
        └──────────────────────────────┘
                        │           ST3
        ┌──────────────────────────────┐
        │  CALCULATE LOCAL MOTION VECTORS│
        │   BASED ON IMAGING INFORMATION │
        └──────────────────────────────┘
                        │           ST4
        ┌──────────────────────────────┐
        │ CALCULATE BODY MOTION INDEX VALUE│
        │   BASED ON IMAGING INFORMATION │
        └──────────────────────────────┘
                        │           ST5
        ┌──────────────────────────────┐
        │   DETERMINE WHETHER OR NOT     │
        │  THERE IS BODY MOTION BASED    │──── THERE IS BODY MOTION
        │    ON IMAGING INFORMATION      │
        │ AND BODY MOTION INDEX VALUE    │
        └──────────────────────────────┘
               THERE IS NO BODY MOTION
                        │                              ST10
                        │                    ┌──────────────────────┐
                        ▼           ST6       │   OUTPUT RESULT        │
        ┌──────────────────────────────┐     │  OF DETERMINATION      │
        │     OUTPUT RESULT OF          │     └──────────────────────┘
        │      DETERMINATION            │                 │        ST11
        └──────────────────────────────┘     ┌──────────────────────┐
                        │           ST7       │      WARNING           │
        ┌──────────────────────────────┐     └──────────────────────┘
        │    BODY MOTION CORRECTION     │                 │
        └──────────────────────────────┘                 │
                        │           ST8                   │
        ┌──────────────────────────────┐                 │
        │          COMBINE              │                 │
        └──────────────────────────────┘                 │
                        │           ST9                   │
        ┌──────────────────────────────┐                 │
        │          DISPLAY              │                 │
        └──────────────────────────────┘                 │
                        │                                 │
                        ▼◄────────────────────────────────┘
                    ( END )
```

# FIG.10

BODY MOTION
INDEX VALUE

8mm

# FIG.11

BODY MOTION
INDEX VALUE

8mm

FIG.12

# FIG.13

START

ST21
LONG-LENGTH IMAGING

ST22
OBTAIN IMAGING INFORMATION

ST23
CALCULATE LOCAL MOTION VECTORS

ST24
CALCULATE BODY MOTION INDEX VALUE

ST25
DETERMINE WHETHER OR NOT
THERE IS BODY MOTION BASED
ON IMAGING INFORMATION
AND BODY MOTION INDEX VALUE

THERE IS BODY MOTION

THERE IS NO BODY MOTION

ST26
OUTPUT RESULT OF
DETERMINATION

ST30
OUTPUT RESULT OF
DETERMINATION

ST27
BODY MOTION CORRECTION

ST31
WARNING

ST28
COMBINE

ST29
DISPLAY

END

FIG.14

**START**

ST41
**LONG-LENGTH IMAGING**

ST42
**CALCULATE LOCAL MOTION VECTORS**

ST43
**CALCULATE BODY MOTION INDEX VALUE**

ST44
**DETERMINE WHETHER OR NOT THERE IS BODY MOTION**

ST45
**SELECT BODY MOTION INDEX VALUE TO BE DISPLAYED**

# FIG.15

ST46
**IS BODY MOTION CORRECTION TO BE CONDUCTED?** — NO

YES

ST47
**SELECT BODY MOTION CORRECTION METHOD**

ST48
**OUTPUT INFORMATION**

ST49
**BODY MOTION CORRECTION**

ST50
**COMBINE**

ST51
**DISPLAY COMBINED IMAGE AND BODY MOTION INDEX VALUE**

ST52
**OUTPUT INFORMATION**

ST53
**WARNING**

ST54
**DISPLAY BODY MOTION INDEX VALUE**

**END**

# FIG.16

| AVERAGE DISPLACEMENT | 8mm |
| THREE DIMENSIONAL | 1.2 |
| ROTATION | 2 DEGREES |
| ENLARGEMENT / REDUCTION | a = 0.2 / d = 0.1 |

# FIG.17

| AVERAGE DISPLACEMENT | 8mm |
| THREE DIMENSIONAL | 1.2 |
| ROTATION | 2 DEGREES |
| ENLARGEMENT / REDUCTION | a = 0.2 / d = 0.1 |

FIG.18

**FIG.19**

START

ST61
LONG-LENGTH IMAGING

ST62
CALCULATE LOCAL
MOTION VECTORS

ST63
CALCULATE BODY
MOTION INDEX VALUES

ST64
DETERMINE PRIMARY BODY
MOTION INDEX VALUE

ST65
DETERMINE WHETHER OR
NOT THERE IS BODY MOTION

ST66
SELECT BODY MOTION INDEX
VALUE TO BE DISPLAYED

ST67
IS BODY MOTION CORRECTION
TO BE CONDUCTED?

YES — ST68
SELECT BODY MOTION
CORRECTION METHOD

ST69
OUTPUT INFORMATION

ST70
BODY MOTION CORRECTION

ST71
COMBINE

ST72
DISPLAY COMBINED IMAGE AND
BODY MOTION INDEX VALUE

NO

ST73
OUTPUT INFORMATION

ST74
WARNING

ST75
DISPLAY BODY
MOTION INDEX VALUE

END

# FIG.20

62

60

61

# FIG.21

62

60

61

FIG.22

# FIG.23

START

ST81
LONG-LENGTH IMAGING

ST82
CALCULATE LOCAL MOTION VECTORS

ST83
CALCULATE BODY MOTION INDEX VALUE

ST84
DETERMINE WHETHER OR NOT THERE IS BODY MOTION

THERE IS BODY MOTION

THERE IS NO BODY MOTION

ST85
OUTPUT RESULT OF DETERMINATION

ST86
BODY MOTION CORRECTION

ST87
COMBINE

ST88
DISPLAY

ST89
OUTPUT RESULT OF DETERMINATION

ST90
GENERATE RETAKE ASSISTING INFORMATION

ST91
DISPLAY RETAKE ASSISTING INFORMATION

ST92
WARNING

END

# FIG.24

64

61
65

A BODY MOTION BETWEEN THE FIRST AND SECOND IMAGES

S1

S2

S3

S4

# FIG.25

64

61

A BODY MOTION BETWEEN THE FIRST AND SECOND IMAGES

S1

S2

S3

S4

# FIG.26

64

61
65

A BODY MOTION BETWEEN
THE FIRST AND SECOND
IMAGES

# FIG.27

64

61
65

PLEASE SECURE
THE KNEES WITH A BELT

S1

S2

S3

S4

67

68

A

**FIG.28**

FIG.29

START

LONG-LENGTH IMAGING — ST101

CALCULATE LOCAL MOTION VECTORS — ST102

CALCULATE BODY MOTION INDEX VALUE — ST103

DETERMINE WHETHER OR NOT THERE IS BODY MOTION — ST104

THERE IS BODY MOTION

THERE IS NO BODY MOTION

OUTPUT RESULT OF DETERMINATION — ST105

BODY MOTION CORRECTION — ST106

COMBINE — ST107

DISPLAY — ST108

OUTPUT RESULT OF DETERMINATION — ST109

GENERATE RETAKE ASSISTING INFORMATION — ST110

DISPLAY RETAKE ASSISTING INFORMATION — ST111

RETAKE CONTROL — ST112

WARNING — ST113

END

# FIG.30

```
              ( START )
                  │
                  ▼        ST121
        ┌─────────────────────┐
        │  LONG-LENGTH IMAGING │
        └─────────────────────┘
                  │
                  ▼        ST122
        ┌─────────────────────┐
        │  CALCULATE LOCAL     │
        │  MOTION VECTORS      │
        └─────────────────────┘
                  │
                  ▼              ST123
    ┌───────────────────────────────┐
    │  CALCULATE BODY MOTION INDEX   │
    │ VALUE OF PARALLEL DISPLACEMENT │
    └───────────────────────────────┘
                  │
                  ▼           ST124
      ┌─────────────────────────┐
      │  DETERMINE WHETHER OR    │
      │ NOT THERE IS BODY MOTION │
      └─────────────────────────┘
                  │
                  ▼             ST125
      ╱─────────────────────────╲  NO
     ╱ IS BODY MOTION CORRECTION  ╲──────────────────┐
     ╲     TO BE CONDUCTED?       ╱                   │
      ╲─────────────────────────╱                     │
             │ YES   ST126                            ▼            ST130
      ┌───────────────────────┐              ┌─────────────────────┐
      │  OUTPUT INFORMATION    │              │  OUTPUT INFORMATION  │
      └───────────────────────┘              └─────────────────────┘
                  │                                   │
                  ▼          ST127                    ▼           ST131
      ┌───────────────────────┐              ┌─────────────────────┐
      │ BODY MOTION CORRECTION │              │      WARNING         │
      └───────────────────────┘              └─────────────────────┘
                  │                                   │
                  ▼          ST128                    ▼           ST132
      ┌───────────────────────┐              ┌─────────────────────┐
      │        COMBINE         │              │  DISPLAY BODY MOTION │
      └───────────────────────┘              │    INDEX VALUE       │
                  │                          └─────────────────────┘
                  ▼             ST129                 │
    ┌───────────────────────────────┐                │
    │ DISPLAY COMBINED IMAGE AND     │                │
    │ BODY MOTION INDEX VALUE        │                │
    └───────────────────────────────┘                │
                  │                                   │
                  ▼◄──────────────────────────────────┘
              (  END  )
```

50

# EP 2 428 164 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009240656 A

**Non-patent literature cited in the description**

- **FUJIO YAMAGUCHI.** Zukei-shori-kogaku. Nikkan Kogyo Shimbun Ltd, 1981, 68-82